(19) **Europäisches Patentamt / European Patent Office / Office européen des brevets**

(11) **EP 3 590 535 A1**

(12) **EUROPEAN PATENT APPLICATION**
published in accordance with Art. 153(4) EPC

(43) Date of publication:
**08.01.2020 Bulletin 2020/02**

(21) Application number: **18761682.6**

(22) Date of filing: **02.03.2018**

(51) Int Cl.:
*A61K 39/395* (2006.01)     *A61K 31/52* (2006.01)
*A61K 31/573* (2006.01)     *A61K 31/706* (2006.01)
*A61K 38/13* (2006.01)       *A61K 45/00* (2006.01)
*A61P 19/00* (2006.01)       *A61P 31/12* (2006.01)
*A61P 35/02* (2006.01)       *A61P 37/06* (2006.01)
*A61P 43/00* (2006.01)

(86) International application number:
**PCT/JP2018/008166**

(87) International publication number:
**WO 2018/159845 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.03.2017   JP 2017039506**

(71) Applicants:
• **St. Marianna University School of Medicine**
  **Kawasaki-shi**
  **Kanagawa 216-8511 (JP)**
• **Kyowa Kirin Co., Ltd.**
  **Chiyoda-ku**
  **Tokyo 1000004 (JP)**

(72) Inventor: **YAMANO Yoshihisa**
  **Kawasaki-shi**
  **Kanagawa 216-8511 (JP)**

(74) Representative: **Hoffmann Eitle**
  **Patent- und Rechtsanwälte PartmbB**
  **Arabellastraße 30**
  **81925 München (DE)**

(54) **PREVENTIVE OR THERAPEUTIC AGENT FOR HTLV-1-ASSOCIATED MYELOPATHY USING LOW-DOSE ANTI-CCR4 ANTIBODY**

(57)     An object of the present invention is to provide a novel therapeutic or preventive agent for human T cell leukemia virus type-1 associated myelopathy (HAM) containing an anti-human CC-chemokine receptor 4 (CCR4) antibody or an antibody fragment thereof as an active ingredient, and characterized by the administration and dosage of the antibody or the antibody fragment thereof. The present invention relates to a therapeutic or preventative agent for HAM characterized in that an anti-human CCR4 antibody or an antibody fragment thereof is contained as an active ingredient and the antibody or the antibody fragment thereof is administered at a low dose.

EP 3 590 535 A1

Printed by Jouve, 75001 PARIS (FR)

**Description**

TECHNICAL FIELD

The present invention relates to a therapeutic or preventive agent for human T cell leukemia virus type-1 (HTLV-1, hereinafter abbreviated to HTLV-1) associated myelopathy (HTLV-1 associated myelopathy: HAM, hereinafter abbreviated to HAM), characterized in that an anti-human CC-chemokine receptor 4 (CCR4) antibody or an antibody fragment thereof is contained as an active ingredient, and the antibody or the antibody fragment thereof is administered at a low dose, and a therapeutic or preventive method for HAM by administering an anti-human CCR4 antibody or an antibody fragment thereof at a low dose.

BACKGROUND ART

HTLV-1 is a retrovirus that chronically infects human T cells. It has been known that while the majority of HTLV-1-infected patients are asymptomatic and can live their lives in good health, approximately 3-5% of the infected persons develop a progressive T-cell malignancy called adult T-cell leukemia (ATL, hereinafter abbreviated to ATL), and another 0.25-3% of the infected persons develop HAM/tropical spastic paraparesis (TSP, hereinafter abbreviated to TSP) (Non-Patent Documents 1-4).

As a main symptom of HAM/TSP (hereinafter also simply referred to as HAM), a motor dysfunction such as lower limb muscle weakness or spasm, sensory disorder, dysuria, or the like is seen (Non-Patent Document 5). Further, some HAM/TSP patients develop as an autoimmune disease characterized by multiorgan lymphocytic infiltration, uveitis, arthritis, polymyositis, Sjogren's syndrome, infective dermatitis, alveolitis, or the like in some cases (Non-Patent Document 6).

It has been reported that in CD4+ CD25+ T cells from the peripheral blood of HAM patients, the expression level of forkhead transcription factor (Foxp3) is lower than those from healthy persons, T cell proliferation regulatory function of CD4+ CD25+ Foxp3+ T cells (regulatory T cells, abbreviated to Treg) is generally reduced, and deterioration in Treg function is caused by HTLV-1 Tax gene (Non-Patent Document 7).

It has been reported that CD4+ CD25+ CCR4+ Foxp3 high T cells are increased in the peripheral blood of ATL patients compared to healthy persons, whereas CD4+ CD25+ CCR4+ Foxp3 low T cells are increased in the peripheral blood of HAM patients compared to healthy persons (Patent Document 1, Non-Patent Document 2). It has been reported that there is a correlation between the number of CD4+ CD25+ CCR4+ Foxp3 low T cells in the peripheral blood and the severity of clinical symptoms of HAM (Patent Document 1). Further, it has been known that a decrease in the amount of HTLV-1 provirus in the peripheral blood is correlated with the long-term prognosis of HAM (Non-Patent Document 8).

Further, it has been reported that in CD4+ CD25+ CCR4+ cells isolated from HAM patients using an anti-human CCR4 antibody, the amount of HTLV-1 proviral DNA is increased compared to CD4+ CD25+ CCR4- cells, and interferon-$\gamma$ (IFN-$\gamma$)+ CD4+ CD25+ Foxp3 low T cells are pathogenic cells of HAM ($T_{HAM}$), and the cells are increased in the peripheral blood of HAM patients (Patent Document 2, Non-Patent Documents 9 and 10).

A hypothesis in which chronic inflammation occurs in HAM patients due to positive feedback that chemokine CXCL10 is produced from stellate cells by IFN-$\gamma$ that HTLV-1-infected cells produce in the central nervous system, and the HTLV-1-infected cells that express CXCR3 serving as a receptor for CXCL10 are recruited into the cerebrospinal fluid (CSF) has been proposed (Non-Patent Document 11).

In the clinical treatment of HAM patients, a treatment with a steroid drug such as prednisolone has been conducted as a treatment of chronic inflammatory response and a treatment with interferon $\alpha$ has been conducted as an anti-viral treatment.

Meanwhile, CCR4 is a seven-transmembrane-type membrane protein that expresses on CD4+ T cells, and thymus and activation-regulated chemokine (TARC)/CCL17 and macrophage-derived chemokine (MDC)/CCL22 are known as its ligands. CCR4 is known to express on Th2, Th17 and Treg cells.

As the anti-human CCR4 antibody, an anti-human CCR4 chimeric antibody (Non-Patent Document 12) and an anti-human CCR4 humanized antibody (Non-Patent Document 13) are known. The anti-human CCR4 humanized antibody [general name: Mogamulizumab, product name: Poteligeo (registered trademark)] was approved for CCR4-positive ATL, relapsed or refractory CCR4-positive peripheral T cell lymphoma, and relapsed or refractory CCR4-positive cutaneous T cell lymphoma, and it is intravenously infused at 1 mg/kg 8 times at intervals of 1 week. Further, when it is used in combination with another antineoplastic agent against CCR4-positive ATL, it is intravenously infused at 1 mg/kg 8 times at intervals of 2 weeks (Non-Patent Document 14).

A therapeutic method for HAM using an anti-human CCR4 antibody is known (Patent Document 3). Further, it is known that a phase II clinical trial for HAM using mogamulizumab has been conducted (Non-Patent Document 11).

CITATION LIST

PATENT DOCUMENTS

> PATENT DOCUMENT 1: JP-A 2010-17130
> PATENT DOCUMENT 2: JP-A 2010-100578
> PATENT DOCUMENT 3: WO 2014/007303

NON-PATENT DOCUMENTS

> NON-PATENT DOCUMENT 1: Uchiyama et al, Blood, 1977; 50: 481-492
> NON-PATENT DOCUMENT 2: Gessain et al, Lancet, 1985; 2: 407-410
> NON-PATENT DOCUMENT 3: Osame et al, Lancet, 1986; 1; 1031-1032
> NON-PATENT DOCUMENT 4: Kaplan et al, J. Aquir. Immune Defi. Syndro., 1990; 3: 1096-1101
> NON-PATENT DOCUMENT 5: Fuzii et al, Life Sciences, 2014; 104: 9-14
> NON-PATENT DOCUMENT 6: Nakagawa et al, J. Neurovirol., 1995; 1: 50-61
> NON-PATENT DOCUMENT 7: Yamano et al, The Journal of Clinical Investigation, 2005; 115: 1361-1368
> NON-PATENT DOCUMENT 8: Olind et al, Arch. Neurol. 2006; 63: 1560-1566
> NON-PATENT DOCUMENT 9: Yamano et al, PLoS One, 2009; 4: e6517
> NON-PATENT DOCUMENT 10: Araya et al, Viruses, 2011; 3: 1532-1548
> NON-PATENT DOCUMENT 11: Yamano et al, Clinical and Experomental Neuroimmunology, 2015: 6; 395-401
> NON-PATENT DOCUMENT 12: Niwa et al, Cancer Res., 2004; 64: 2127-2133
> NON-PATENT DOCUMENT 13: Ishii et al, Clin. Cancer Res., 2010; 16: 1520-1531
> NON-PATENT DOCUMENT 14: "Poteligeo (registered trademark), intravenous infusion 20 mg, package insert" revised on May 2015

SUMMARY OF INVENTION

TECHNICAL PROBLEM

As described above, a therapeutic method for HAM using an anti-human CCR4 antibody is known, however, clinical efficacy when it is administered to humans has not been validated, and it was not clear what administration and dosage of the anti-human CCR4 antibody should be used for the treatment of HAM patients, or what therapeutic effect on HAM can be achieved.

Accordingly, an object of the present invention is to provide a novel therapeutic or preventive agent for HAM containing an anti-human CCR4 antibody or an antibody fragment thereof as an active ingredient, and characterized by the administration and dosage of the antibody or the antibody fragment thereof.

SOLUTION TO PROBLEM

The present inventors found that by administering a low dose of an anti-human CCR4 antibody to a HAM patient, a remarkable therapeutic effect on HAM appears, and thus completed the present invention. That is, the invention of the present application relates to the following (1) to (42).

(1) A preventive or therapeutic agent for HAM, comprising:

> an anti-human CCR4 antibody or an antibody fragment thereof as an active ingredient,
> wherein the antibody or the antibody fragment thereof is administered at a low dose.

(2) The preventive or therapeutic agent for HAM according to (1),
wherein the anti-human CCR4 antibody or the antibody fragment thereof is administered at a dose of 1 mg/kg or less at administration intervals of 4 weeks or more.
(3) The preventive or therapeutic agent for HAM according to (1) or (2),

wherein the anti-human CCR4 antibody or the antibody fragment thereof is administered at a dose of 0.3 mg/kg at administration intervals of 12 weeks.

(4) The preventive or therapeutic agent for HAM according to any one of (1) to (3),
wherein the anti-human CCR4 antibody or the antibody fragment thereof is an anti-human CCR4 antibody or an antibody fragment thereof comprising an antibody heavy chain variable region (hereinafter, abbreviated to VH) comprising complementarily determining regions (hereinafter, abbreviated to CDRs) 1, 2, and 3 comprising amino acid sequences represented by SEQ ID NOS: 1, 2, and 3, respectively, and an antibody light chain variable region (hereinafter, abbreviated to VL) comprising CDRs 1, 2, and 3 comprising amino acid sequences represented by SEQ ID NOS: 4, 5, and 6, respectively.

(5) The preventive or therapeutic agent for HAM according to any one of (1) to (4),
wherein the anti-human CCR4 antibody or the antibody fragment thereof is an anti-human CCR4 antibody or an antibody fragment thereof comprising VH comprising an amino acid sequence represented by SEQ ID NO: 7 and VL comprising an amino acid sequence represented by SEQ ID NO: 8.

(6) The preventive or therapeutic agent for HAM according to any one of (1) to (5), wherein the anti-human CCR4 antibody is mogamulizumab.

(7) The preventive or therapeutic agent for HAM according to any one of (1) to (6),
wherein the antibody fragment is Fab, Fab', $F(ab')_2$, scFv, or a CDR-containing peptide.

(8) The preventive or therapeutic agent for HAM according to any one of (1) to (7), wherein an immunosuppressant is used in combination.

(9) The preventive or therapeutic agent for HAM according to (8),
wherein the immunosuppressant is administered at a low dose.

(10) The preventive or therapeutic agent for HAM according to (8) or (9),
wherein the immunosuppressant is any one immunosuppressant selected from prednisolone, methylprednisolone, dexamethasone, betamethasone, azathioprine, cyclosporine, tacrolimus, a JAK inhibitor, and an NF-κB inhibitor.

(11) The preventive or therapeutic agent for HAM according to any one of (1) to (10),
wherein the preventive or therapeutic agent decreases at least any one biomarker selected from the amount of HTLV-1 proviral DNA in the peripheral blood, the amount of HTLV-1 proviral DNA in the CSF, and the number of cells in CSF of a HAM patient.

(12) The preventive or therapeutic agent for HAM according to any one of (1) to (11),
wherein the preventive or therapeutic agent decreases the amount of at least one of neopterin and CXCL10 in CSF of a HAM patient.

(13) The preventive or therapeutic agent for HAM according to any one of (1) to (12),
wherein the preventive or therapeutic agent prevents deterioration of the motor function of a HAM patient or improves the motor function.

(14) The preventive or therapeutic agent for HAM according to (13),
wherein the motor function is evaluated by at least one of Modified Ashworth Scale and Osame's motor disability score.

(15) A preventive or therapeutic method for HAM, comprising:
administering an anti-human CCR4 antibody or an antibody fragment thereof at a low dose.

(16) The preventive or therapeutic method for HAM according to (15), comprising:
administering the anti-human CCR4 antibody or the antibody fragment thereof at a dose of 1 mg/kg or less at administration intervals of 4 weeks or more.

(17) The preventive or therapeutic method for HAM according to (15) or (16), comprising:
administering the anti-human CCR4 antibody or the antibody fragment thereof at a dose of 0.3 mg/kg at administration intervals of 12 weeks.

(18) The preventive or therapeutic method for HAM according to any one of (15) to (17),
wherein the anti-human CCR4 antibody or the antibody fragment thereof is an anti-human CCR4 antibody or an antibody fragment thereof comprising VH comprising CDRs 1, 2, and 3 comprising amino acid sequences represented by SEQ ID NOS: 1, 2, and 3, respectively, and VL comprising CDRs 1, 2, and 3 comprising amino acid sequences represented by SEQ ID NOS: 4, 5, and 6, respectively.

(19) The preventive or therapeutic method for HAM according to any one of (15) to (18),
wherein the anti-human CCR4 antibody or the antibody fragment thereof is an anti-human CCR4 antibody or an antibody fragment thereof comprising VH comprising an amino acid sequence represented by SEQ ID NO: 7 and VL comprising an amino acid sequence represented by SEQ ID NO: 8.

(20) The preventive or therapeutic method for HAM according to any one of (15) to (19),
wherein the anti-human CCR4 antibody is mogamulizumab.

(21) The preventive or therapeutic method for HAM according to any one of (15) to (20),
wherein the antibody fragment is Fab, Fab', $F(ab')_2$, scFv, or a CDR-containing peptide.

(22) The preventive or therapeutic method for HAM according to any one of (15) to (21), comprising:
using an immunosuppressant in combination.
(23) The preventive or therapeutic method for HAM according to (22), comprising:
administering the immunosuppressant at a low dose.
(24) The preventive or therapeutic method for HAM according to (22) or (23),
wherein the immunosuppressant is any one immunosuppressant selected from prednisolone, methylprednisolone, dexamethasone, betamethasone, azathioprine, cyclosporine, tacrolimus, a JAK inhibitor, and an NF-κB inhibitor.
(25) The preventive or therapeutic method for HAM according to any one of (15) to (24),
wherein the preventive or therapeutic method decreases at least any one biomarker selected from the amount of HTLV-1 proviral DNA in the peripheral blood, the amount of HTLV-1 proviral DNA in CSF, and the number of cells in CSF of a HAM patient.
(26) The preventive or therapeutic method for HAM according to any one of (15) to (25),
wherein the preventive or therapeutic method decreases the amount of at least one of neopterin and CXCL10 in CSF of a HAM patient.
(27) The preventive or therapeutic method for HAM according to any one of (15) to (26),
wherein the preventive or therapeutic method prevents deterioration of the motor function of a HAM patient or improves the motor function.
(28) The preventive or therapeutic method for HAM according to (27),
wherein the motor function is evaluated by at least one of Modified Ashworth Scale and Osame's motor disability score.
(29) An anti-human CCR4 antibody or an antibody fragment thereof for use in treatment or prevention for HAM, wherein the antibody or the antibody fragment thereof is administered at a low dose.
(30) Use of an anti-human CCR4 antibody or an antibody fragment thereof for producing a preventive agent or a therapeutic agent for HAM,
wherein the preventive agent or the therapeutic agent is administered at a low dose.
(31) A method for preventing deterioration of the motor function of a HAM patient and/or a method for improving the motor function, comprising:
administering an anti-human CCR4 antibody or an antibody fragment thereof at a low dose.
(32) The method for preventing deterioration of the motor function of a HAM patient and/or the method for improving the motor function according to (31), comprising:
administering the anti-human CCR4 antibody or the antibody fragment thereof at a dose of 1 mg/kg or less at administration intervals of 4 weeks or more.
(33) The method for preventing deterioration of the motor function of a HAM patient and/or the method for improving the motor function according to (31) or (32), comprising:
administering the anti-human CCR4 antibody or the antibody fragment thereof at a dose of 0.3 mg/kg at administration intervals of 12 weeks.
(34) The method for preventing deterioration of the motor function of a HAM patient and/or the method for improving the motor function according to any one of (31) to (33),
wherein the anti-human CCR4 antibody or the antibody fragment thereof is an anti-human CCR4 antibody or an antibody fragment thereof comprising VH comprising CDRs 1, 2, and 3 comprising amino acid sequences represented by SEQ ID NOS: 1, 2, and 3, respectively, and VL comprising CDRs 1, 2, and 3 comprising amino acid sequences represented by SEQ ID NOS: 4, 5, and 6, respectively.
(35) The method for preventing deterioration of the motor function of a HAM patient and/or the method for improving the motor function according to any one of (31) to (34),
wherein the anti-human CCR4 antibody or the antibody fragment thereof is an anti-human CCR4 antibody or an antibody fragment thereof comprising VH comprising an amino acid sequence represented by SEQ ID NO: 7 and VL comprising an amino acid sequence represented by SEQ ID NO: 8.
(36) The method for preventing deterioration of the motor function of a HAM patient and/or the method for improving the motor function according to any one of (31) to (35),
wherein the anti-human CCR4 antibody is mogamulizumab.
(37) The method for preventing deterioration of the motor function of a HAM patient and/or the method for improving the motor function according to any one of (31) to (36),
wherein the antibody fragment is Fab, Fab', F(ab')$_2$, scFv, or a CDR-containing peptide.
(38) The method for preventing deterioration of the motor function of a HAM patient and/or the method for improving the motor function according to any one of (31) to (37), comprising:
using an immunosuppressant in combination.
(39) The method for preventing deterioration of the motor function of a HAM patient and/or the method for improving the motor function according to (38), comprising:

administering the immunosuppressant at a low dose.

(40) The method for preventing deterioration of the motor function of a HAM patient and/or the method for improving the motor function according to (38) or (39),

wherein the immunosuppressant is any one immunosuppressant selected from prednisolone, methylprednisolone, dexamethasone, betamethasone, azathioprine, cyclosporine, tacrolimus, a JAK inhibitor, and an NF-κB inhibitor.

(41) The method for preventing deterioration of the motor function of a HAM patient and/or the method for improving the motor function according to any one of (31) to (40),

wherein the method decreases at least any one biomarker selected from the amount of HTLV-1 proviral DNA in the peripheral blood, the amount of HTLV-1 proviral DNA in CSF, and the number of cells in CSF of a HAM patient.

(42) The method for preventing deterioration of the motor function of a HAM patient and/or the method for improving the motor function according to any one of (31) to (41),

wherein the method decreases the amount of at least one of neopterin and CXCL10 in CSF of a HAM patient.

ADVANTAGEOUS EFFECTS OF INVENTION

According to the present invention, a therapeutic or preventive agent and a therapeutic or preventive method for HAM including an anti-human CCR4 antibody or an antibody fragment thereof as an active ingredient, and exhibiting a remarkable therapeutic effect on HAM by administering the antibody or the antibody fragment thereof at a low dose can be provided.

BRIEF DESCRIPTION OF DRAWINGS

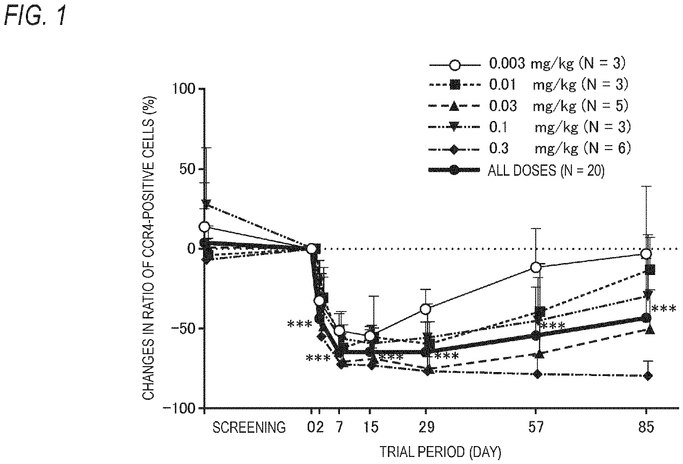

[Fig. 1] Fig. 1 shows changes in the ratio of CCR4-positive cells in peripheral blood mononuclear cells (hereinafter abbreviated to PBMCs) of HAM patients in a phase I trial. The vertical axis represents the percentage (%) of the change in the ratio of CCR4-positive cells in PBMCs using the value on the day before administering an anti-human CCR4 humanized antibody mogamulizumab (Day 0) as a reference value by an average for each dose level, and the horizontal axis represents the day when PBMCs were collected from the patients. The error bars indicate the standard deviations. In comparison with the reference value, a paired T-test was used ($*p<0.05$, $**p<0.01$, $***p<0.001$).

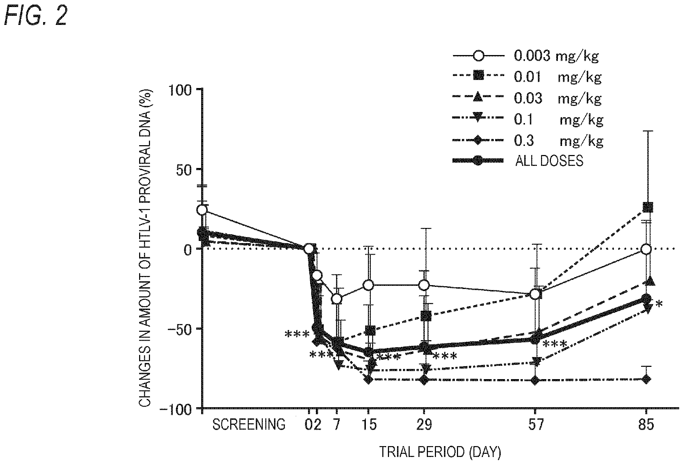

[Fig. 2] Fig. 2 shows changes in the amount of HTLV-1 proviral DNA in PBMCs of HAM patients in a phase I trial. The vertical axis represents the percentage (%) of the change in the amount of HTLV-1 proviral DNA in PBMCs using the value on Day 0 as a reference value by an average for each dose level, and the horizontal axis represents the day when PBMCs were collected from the patients. The error bars indicate the standard deviations. In comparison with the reference value, a paired T-test was used ($*p<0.05$, $**p<0.01$, $***p<0.001$).

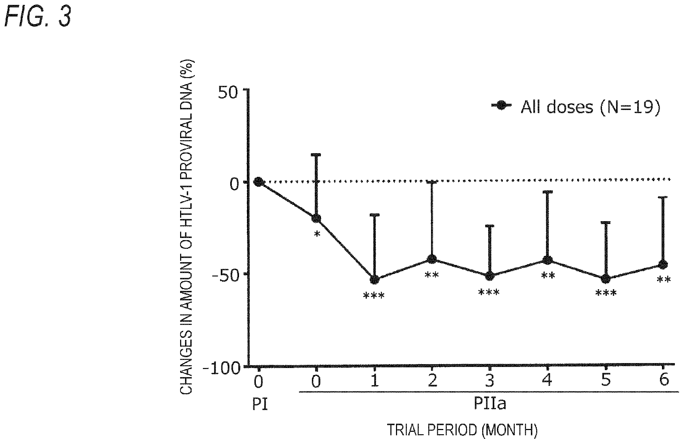

[Fig. 3] Fig. 3 shows changes in the amount of HTLV-1 proviral DNA in PBMCs of HAM patients in a phase IIa trial. The vertical axis represents the percentage (%) of the change in the amount of HTLV-1 proviral DNA in PBMCs using the value on Day 0 in the phase I as a reference value by an average for all patients, and the horizontal axis represents the month when PBMCs were collected from the patients. The error bars indicate the standard deviations. In comparison with the reference value, a paired T-test was used ($*p<0.05$, $**p<0.01$, $***p<0.001$).

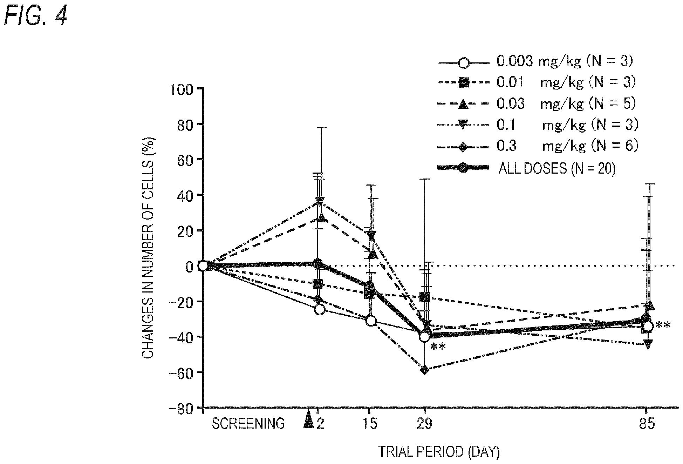

[Fig. 4] Fig. 4 shows changes in the number of cells in CSF of HAM patients in a phase I trial. The vertical axis represents the percentage (%) of the change in the number of cells in CSF using the value at the time of screening as a reference value by an average for each dose level, and the horizontal axis represents the day when CSF was collected from the patients. The black arrowhead on the horizontal axis indicates the day when mogamulizumab was administered (Day 1). The error bars indicate the standard deviations. In comparison with the reference value, a paired T-test was used ($*p<0.05$, $**p<0.01$, $***p<0.001$).

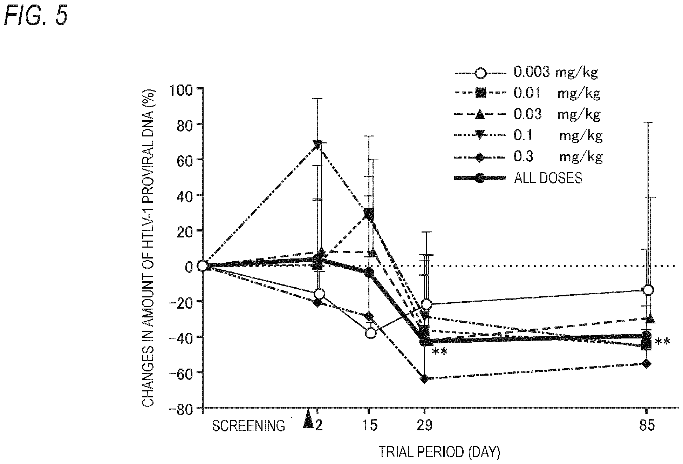

[Fig. 5] Fig. 5 shows changes in the amount of HTLV-1 proviral DNA per 1 mL of CSF of HAM patients in a phase I trial. The vertical axis represents the percentage (%) of the change in the amount of HTLV-1 proviral DNA per 1 mL of CSF using the value at the time of screening in the phase I as a reference value by an average for each dose level, and the horizontal axis represents the day when CSF was collected from the patients. The black arrowhead on the horizontal axis indicates the day when mogamulizumab was administered (Day 1). The error bars indicate the standard deviations. In comparison with the reference value, a paired T-test was used ($*p<0.05$, $**p<0.01$, $***p<0.001$).

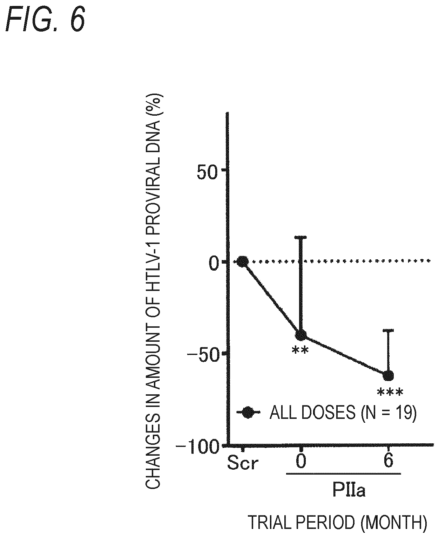

[Fig. 6] Fig. 6 shows changes in the amount of HTLV-1 proviral DNA per 1 mL of CSF of HAM patients in a phase IIa trial. The vertical axis represents the percentage (%) of the change in the amount of HTLV-1 proviral DNA per 1 mL of CSF using the value at the time of screening in the phase I as a reference value by an average for all patients, and the horizontal axis represents the month when CSF was collected from the patients. "Scr" indicates when the screening was performed. The error bars indicate the standard deviations. In comparison with the reference value, a paired T-test was used (*p<0.05, **p<0.01, ***p<0.001).

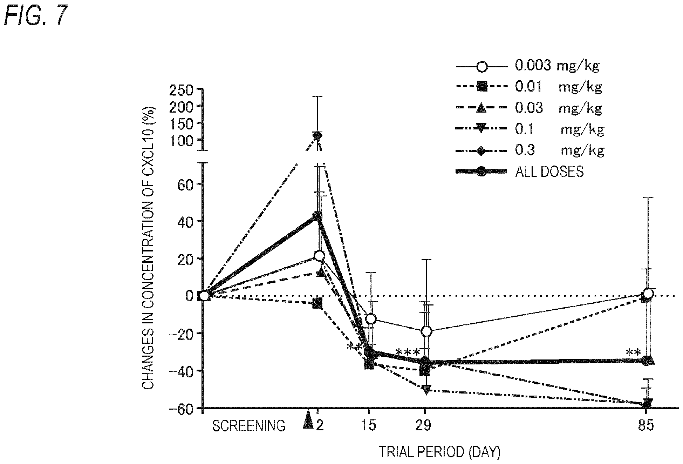

[Fig. 7] Fig. 7 shows changes in the concentration of CXCL10 in CSF of HAM patients in a phase I trial. The vertical axis represents the percentage (%) of the change in the concentration of CXCL10 in CSF using the value at the time of screening as a reference value by an average for each dose level, and the horizontal axis represents the day when CSF was collected from the HAM patients. The black arrowhead on the horizontal axis indicates Day 1. The error bars indicate the standard deviations. In comparison with the reference value, a paired T-test was used (*p<0.05, **p<0.01, ***p<0.001).

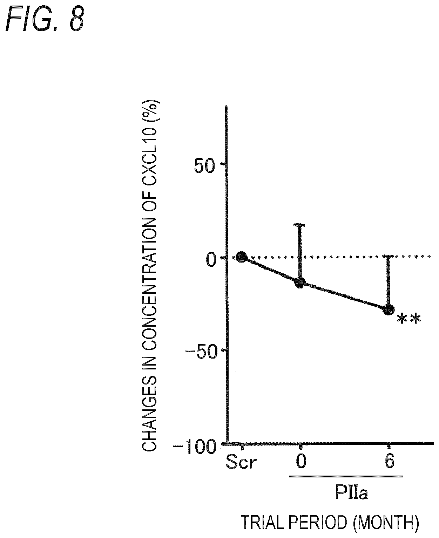

[Fig. 8] Fig. 8 shows changes in the concentration of CXCL10 in CSF of HAM patients in a phase IIa trial. The vertical axis represents the percentage (%) of the change in the concentration of CXCL10 in CSF using the value at the time of screening in the phase I as a reference value by an average for all patients, and the horizontal axis represents the month when CSF was collected from the patients. "Scr" indicates when the screening was performed. The error bars indicate the standard deviations. In comparison with the reference value, a paired T-test was used (*p<0.05, **p<0.01, ***p<0.001).

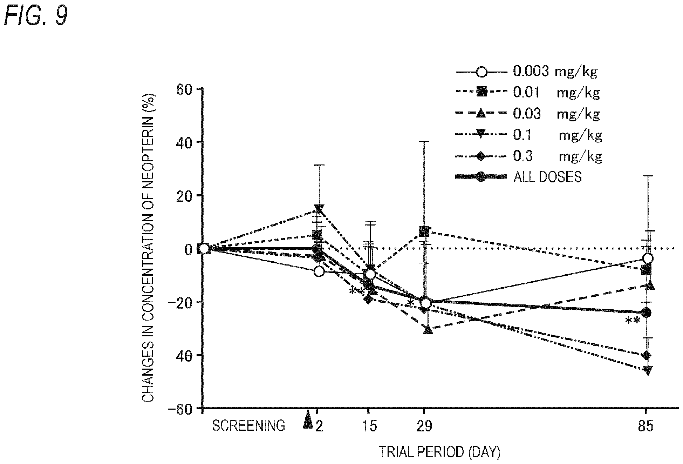

[Fig. 9] Fig. 9 shows changes in the concentration of neopterin in CSF of HAM patients in a phase I trial. The vertical axis represents the percentage (%) of the change in the concentration of neopterin in CSF using the value at the time of screening as a reference value by an average for each dose level, and the horizontal axis represents the day when CSF was collected from the patients. The black arrowhead on the horizontal axis indicates Day 1. The error bars indicate the standard deviations. In comparison with the reference value, a paired T-test was used (*p<0.05, **p<0.01, ***p<0.001).

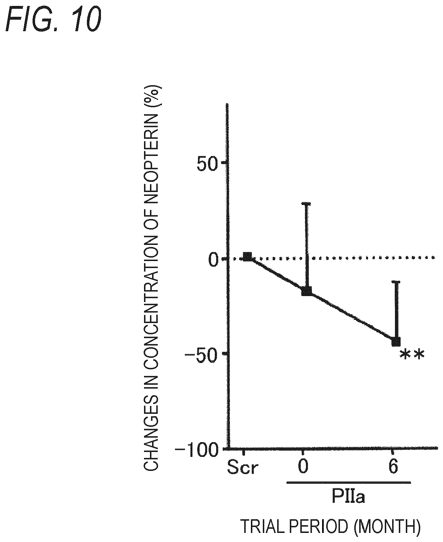

[Fig. 10] Fig. 10 shows changes in the concentration of neopterin in CSF of HAM patients in a phase IIa trial. The vertical axis represents the percentage (%) of the change in the concentration of neopterin in CSF using the value at the time of screening in the phase I as a reference value by an average for all patients, and the horizontal axis represents the month when CSF was collected from the patients. "Scr" indicates when the screening was performed. The error bars indicate the standard deviations. In comparison with the reference value, a paired T-test was used (*p<0.05, **p<0.01, ***p<0.001).

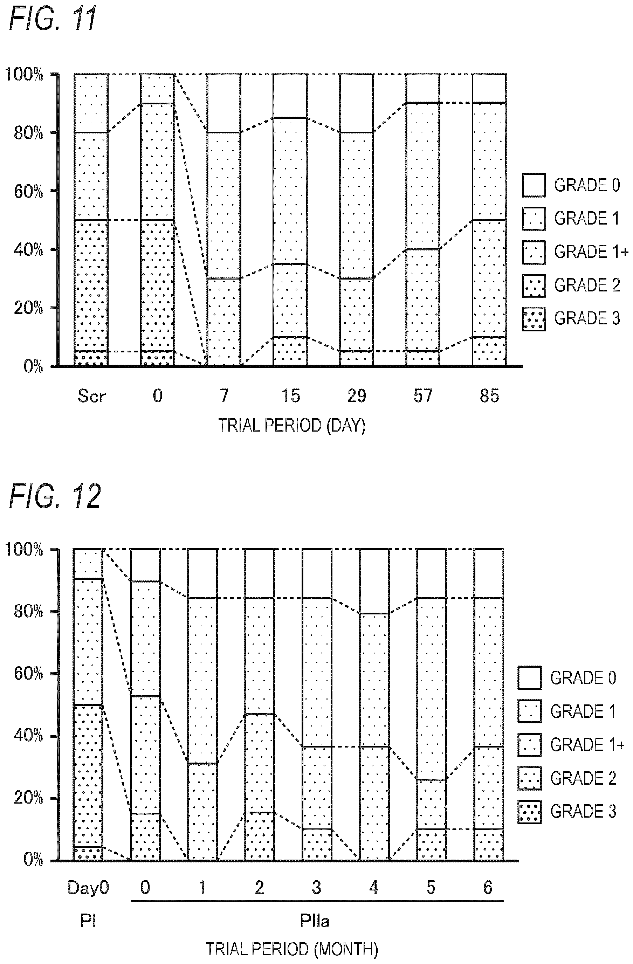

[Fig. 11] Fig. 11 shows changes in grades of Modified Ashworth Scale (hereinafter sometimes abbreviated to MAS) of HAM patients in a phase I trial. The vertical axis represents the ratio (%) of the patients in each grade when the total number of patients is taken as 100%. The horizontal axis represents the day when the evaluation was performed. "Scr" indicates when the screening was performed.

[Fig. 12] Fig. 12 shows changes in grades of MAS of HAM patients in a phase IIa trial. The vertical axis represents the ratio (%) of the patients in each grade when the total number of patients is taken as 100%. The horizontal axis represents the month when the evaluation was performed.

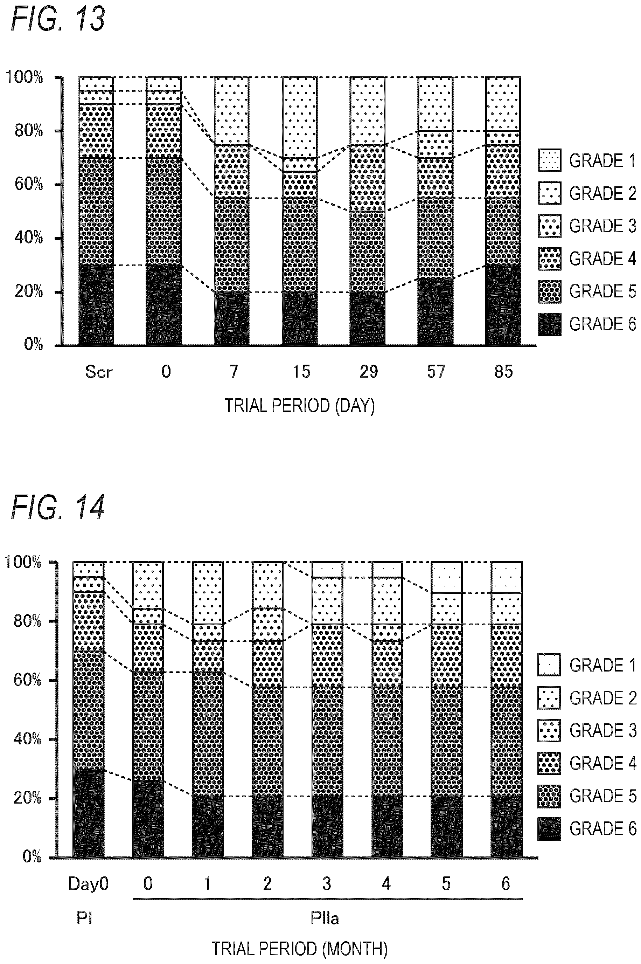

[Fig. 13] Fig. 13 shows changes in grades of Osame's motor disability score (hereinafter sometimes abbreviated to OMDS) of HAM patients in a phase I trial. The vertical axis represents the ratio (%) of the patients in each grade when the total number of patients is taken as 100%. The horizontal axis represents the day when the evaluation was performed. "Scr" indicates when the screening was performed.

[Fig. 14] Fig. 14 shows changes in grades of OMDS of HAM patients in a phase IIa trial. The vertical axis represents the ratio (%) of the patients in each grade when the total number of patients is taken as 100%. The horizontal axis represents the month when the evaluation was performed.

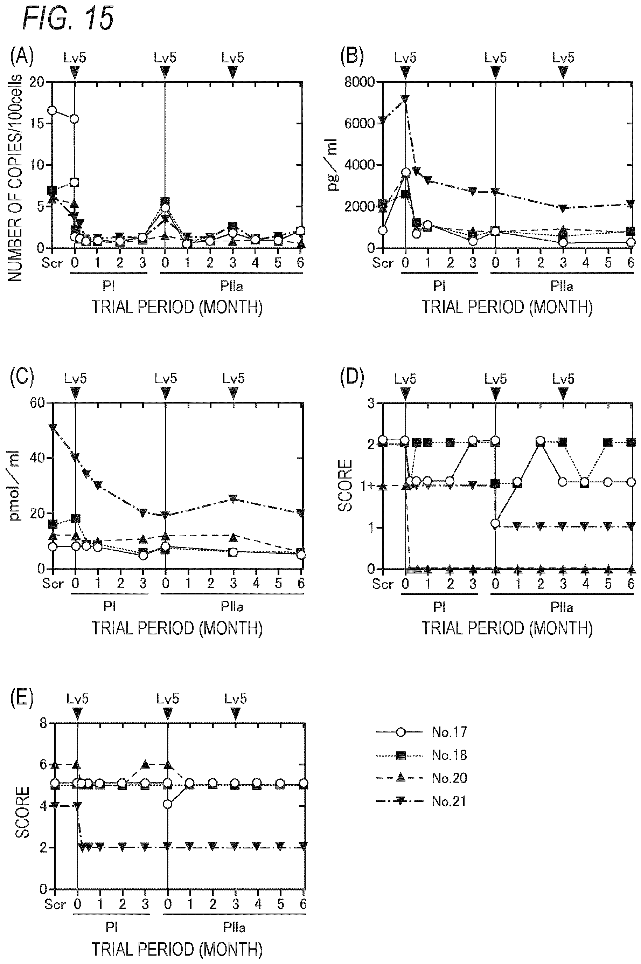

[Fig. 15] Figs. 15(A) to (E) show shifts of the amount of proviral DNA in PBMCs, the concentration of CXCL10 in CSF, the concentration of neopterin in CSF, MAS, and OMDS with respect to four HAM patients to whom 0.3 mg/kg mogamulizumab was administered every three months during a phase IIa trial period for each patient. In Fig. 15(A), the vertical axis represents the proviral DNA copy number per 100 PBMCs, and the horizontal axis represents the month when PBMCs were collected. In Fig. 15(B), the vertical axis represents the concentration (pg/mL) of CXCL10 in CSF, and the horizontal axis represents the month when CSF was collected. In Fig. 15(C), the vertical axis represents the concentration (pg/mL) of neopterin in CSF, and the horizontal axis represents the month when CSF was collected. In Fig. 15(D), the vertical axis represents the score of MAS, and the horizontal axis represents the month when the evaluation was performed. In Fig. 15(E), the vertical axis represents the score of OMDS, and the horizontal axis represents the month when the evaluation was performed. In the view of each of Figs. 15(A) to (E), the black arrowheads on the upper side of the graphs indicate the timing of administration of mogamulizumab.

DESCRIPTION OF EMBODIMENTS

The present invention relates to a therapeutic or preventive agent for HAM characterized in that an anti-human CCR4 antibody or an antibody fragment thereof is contained as an active ingredient, and the antibody or the antibody fragment thereof is administered at a low dose, and a therapeutic or preventive method for HAM by administering an anti-human CCR4 antibody or an antibody fragment thereof at a low dose.

Further, the present invention relates to a method for preventing deterioration of the motor function of a HAM patient, a method for improving the motor function of a HAM patient, and a method for improving the severity of HAM in a HAM patient, characterized by administering an anti-human CCR4 antibody or an antibody fragment thereof at a low dose. The severity of HAM is the severity of pathological condition determined based on the clinical symptoms (motor function, urination function, neurologic symptoms, etc.) or immunological symptoms (a cell population in which a specific marker is expressed, the amount of cytokine, etc.) of a HAM patient described below.

HTLV-1 is a retrovirus that chronically infects human T cells. It has been known that while the majority of HTLV-1-infected patients are asymptomatic and can live their lives in good health, 0.25-3% of the infected persons develop HAM/TSP.

HAM is a refractory neurological disease having a pathological feature of chronic myelitis caused by infiltration of peripheral blood HTLV-1-infected T cells into the spinal cord. As the route of infection with HTLV-1, vertical transmission from mother to child, and horizontal transmission through blood transfusion and sexual contact are known. Examples of the symptoms of HAM include motor disorder, dysuria, neurological disorder, and the like caused by the disturbance of the pyramidal tract running in the lateral funiculus of the thoracic spinal cord.

In the present invention, a HAM patient and an asymptomatic HTLV-1 carrier (AC, hereinafter sometimes abbreviated to AC, and also referred to as HTLV-1 inapparent infected person) are infected with HTLV-1 virus, and an anti-HTLV-1 antibody is detected in the peripheral blood or CSF compared to a normal healthy person (healthy person).

In the present invention, in a HAM patient, an increase in the anti-HTLV-1 antibody titer, an increase in the amount of HTLV-1 proviral DNA, an increase in the amount of HTLV-1 Tax mRNA, and an increase in activated CD4+ cells (CD4+ CD25+ T cells) in the peripheral blood or CSF, and an increase in the concentration of neopterin in CSF accompanying the finding of inflammation in the spinal region, and the like compared to an AC and a healthy person are observed. Therefore, the HAM patient is distinguished from the AC and the healthy person.

In the present invention, the AC refers to a patient in whom infection with HTLV-1 virus has been established, but clinical symptoms are not observed. Infection with HTLV-1 virus can be determined by whether or not the anti-HTLV-1 antibody titer is present in the peripheral blood of a patient.

In the present invention, the treating HAM means suppressing deterioration of the symptoms of HAM or improving the symptoms of HAM, or the like. By suppressing deterioration of the symptoms of HAM or improving the symptoms of HAM, the long-term prognosis of a HAM patient can be improved, and as a result, the transition to motor dysfunction such as a wheelchair-bound state or a bedridden state can be prevented.

In the present invention, the preventing HAM means decreasing the risk of the onset of HAM in the AC, or the like.

In the present invention, examples of the symptoms of HAM include deterioration of motor function, dysuria, and sensory disorder.

The deterioration of motor function refers to the occurrence of disorder in voluntary motor function, and refers to, for example, the following: movement such as walking or running by oneself becomes slow or one cannot move around.

In the present invention, the motor function can be evaluated by a walking test for a 10 m walking time, a 2 min walking distance or a 6 min walking distance, or the like, evaluation of lower limb clonus, the Time up and go test (D. Podsiadlo et al, Journal of American Geriatrics Society: 1991; 39, 142-148) for evaluating a functional mobility, Modified Ashworth Scale (hereinafter sometimes abbreviated to MAS) [R. W. Bohannon et al, Physigcal Therapy: 1987; 67(2), 206-207] showing the levels of spasticity, or Osame's motor disability score (hereinafter sometimes abbreviated to OMDS) [S. Izumo et al, Neurology: 1996; 46(4): 1016-1021] showing comprehensive motor function.

When the MAS is improved in patients for whom rehabilitation could hardly be carried out due to spasm, rehabilitation can be carried out for the patients, leading to improvement of a walking state or improvement of a daily living activity in the end. Further, the maintaining the MAS score without increasing the score indicates that the long-term prognosis of the HAM patient is improved.

The OMDS is known to well reflect the progress of the pathological state of HAM. That is, a decrease in OMDS score reflects an improvement of the disease. Further, the maintaining the OMDS score without increasing the score indicates that the long-term prognosis of the HAM patient is improved.

In the present invention, the preventing deterioration of motor function refers to preventing deterioration of motor function over time by administering a low dose of an anti-human CCR4 antibody or an antibody fragment thereof so as to maintain the state of motor function before the start of administration. Specifically, for example, at least one of maintaining a time for walking a given distance, maintaining a walking distance for a given time, maintaining a state in which the MAS score is not increased, and maintaining a state in which the OMDS score is not increased is exemplified.

Further, in the present invention, the improving motor function refers to improving the state of motor function compared to that before the start of administration by administering a low dose of an anti-human CCR4 antibody or an antibody fragment thereof. Specifically, for example, at least one of reducing a time for walking a given distance, increasing a walking distance for a given time, decreasing the MAS score, and decreasing the OMDS score is exemplified.

In the present invention, the dysuria refers to urine collection disorder, urination disorder, or the like, and also includes a complication thereof. Specifically, for example, frequent urination, urge urinary incontinence, difficulty in urination, etc. are exemplified.

In the present invention, the dysuria can be evaluated by, for example, International Prostate Symptom Score (I-PSS), Overactive Bladder Symptom Score (OABSS), International Consultation on Incontinence Questionnaire-Short Form, Nocturia-Quality of Life Questionnaire (N-QOL), or the like.

In the present invention, the sensory disorder refers to lower limb numbness, lower limb pain, or the like. In the present invention, the sensory disorder can be evaluated using, for example, Visual Analogue Scale (hereinafter abbreviated to VAS).

The HAM affects not only motor disorder or dysuria, but also daily life due to various symptoms. As one embodiment of the present invention, a therapeutic or preventive method and a therapeutic or preventive agent for HAM including improving such a general condition by administering a low dose of an anti-human CCR4 antibody or an antibody fragment thereof to a HAM patient are exemplified. In the present invention, the general condition of HAM can be evaluated by, for example, VAS.

As one embodiment of the present invention, a therapeutic or preventive method and a therapeutic or preventive agent characterized by targeting HTLV-1-infected cells in at least one of the peripheral blood and CSF of a HAM patient are exemplified.

In the present invention, as the HTLV-1-infected cells, for example, CD4+T cells, CCR4+ T cells, CD4+ CD25+ T cells, CD4+ CD25+ Foxp3 low T cells, CD4+ CD25+ CCR4+ T cells, CD4+ CD25+ CCR4+ Foxp3 low T cells, CD4+ CD25+ CCR4+ Foxp3 low IFN-$\gamma$+ T cells (T$_{HAM}$), CD8+ CCR4+ T cells, etc. are exemplified.

Preferably, any one selected from CD4+ CD25+ CCR4+ T cells, CD4+ CD25+ CCR4+ Foxp3 low T cells, and CD4+ CD25+ CCR4+ Foxp3 low IFN-$\gamma$+ T cells which are CCR4+ T cells is exemplified, more preferably CD4+ CD25+ CCR4+ Foxp3 low IFN-$\gamma$+ T cells (T$_{HAM}$) are exemplified.

In the peripheral blood of a HAM patient, cytotoxic CD8+ T cells, which are specific to HTLV-1 transactivator protein Tax, are increased compared to those in an asymptomatic HTLV-1 carrier and a healthy person, resulting in causing chronic inflammation in HTLV-1 -infected tissues (Yamano et al, Blood, 2002; 99: 88-94). Therefore, CD8+ CCR4+ T cells are exemplified as target cells for the therapeutic or preventive method and the therapeutic or preventive agent of the present invention.

Further, as the HTLV-1-infected cells targeted by the therapeutic or preventive method and the therapeutic or preventive agent of the present invention, CD4+ CD25+ T cells among the HTLV-1-infected cells in the peripheral blood of a HAM patient are exemplified (Yamano et al, J. Exp. Med., 2004; 199; 1367-1377).

Among the peripheral blood cells of an HTLV-1-infected person, CD4+ CD25+ T cells are reservoir cells for HTLV-1, and when regulatory T cells (hereinafter, abbreviated to Treg) contained in a CD4+ CD25+ T cell fraction and regulated by the expression of Foxp3 are infected with HTLV-1, the expression level of Foxp3 is decreased in a Tax-dependent manner, and the regulatory function of the T cells is decreased or lost (Yamano et al, J. Clin Invest., 2005; 115: 1361-1368). Therefore, the CD4+ CD25+ Foxp3 low T cells are also included as the target cells of the therapeutic or preventive method and the therapeutic or preventive agent of the present invention.

As the HTLV-1-infected cells targeted by the therapeutic or preventive method and the therapeutic or preventive agent of the present invention, CCR4+ T cells and CD4+ CD25+ CCR4+ T cells having an increased amount of HTLV-1 proviral DNA among the HTLV-1-infected cells in the peripheral blood of a HAM patient are exemplified.

Further, CCR4+ T cells, CD4+ CD25+ CCR4+ T cells, and CD4+ CD25+ CCR4+ Foxp3 low T cells are also included as the target cells of the therapeutic or preventive method and the therapeutic or preventive agent of the present invention because the expression of Foxp3 is decreased, the expression of interferon-$\gamma$ (IFN-$\gamma$) is increased, and the expression of interleukin (IL)-2, IL-4, IL-10, and IL-17 is decreased specifically in HTLV-1-infected CD4+ CD25+ CCR4+ T cells (Yamano et al, PLoS One, 2009; 4; e6517).

Further, the ratio of CD4+ CD25+ CCR4+ IFN-$\gamma$+ T cells in PBMCs of a HAM patient and the amount of neopterin correlated with the finding of inflammation in the spinal region of a HAM patient or the severity of HAM show a positive correlation with each other. Meanwhile, there is a low correlation between the amount of HTLV-1 proviral DNA in the peripheral blood of a HAM patient and the amount of neopterin or the severity of HAM, and therefore, an increase in the number of HTLV-1-infected T cells having functional changes such as an increase in the production amount of IFN-$\gamma$ rather than the absolute amount of HTLV-1-infected T cells in the peripheral blood of a patient is more associated with the severity of HAM.

Therefore, as the HTLV-1-infected cells that can become target cells of the therapeutic or preventive method and the therapeutic or preventive agent of the present invention, T cells with a characteristic phenotype of CD4+ CD25+ CCR4+ Foxp3 low IFN-$\gamma$+, namely, pathogenic cells of HAM (hereinafter, sometimes abbreviated to T$_{HAM}$) (Araya et al, Viruses, 2011; 3: 1532-1548) are exemplified.

In the present invention, CD4+, CD8+, CD25+, CCR4+, or IFN-$\gamma$+ cells refer to a cell population that shows substantially a higher fluorescence intensity than a fluorescence intensity caused by a negative control antibody when performing an analysis with a flow cytometer (hereinafter, sometimes abbreviated to FCM) using an antibody specifically binds to each molecule.

Specifically, in the case of a cell membrane protein, the cells can be directly stained with an antibody specific to each antigen molecule, and in the case of a secretory protein, the cells can be stained by performing a membrane permeation treatment using an appropriate surfactant or the like, and a protein fixation treatment.

In the present invention, the Foxp3 low cells indicate cells having reduced Foxp3 expression. The cells having reduced Foxp3 expression indicate the Foxp3 expression level comparable to that of CD4+ CD25+ CD45RO- cells and can be selected by comparing the Foxp3 expression level to that of the cell population. Further, the Foxp3 low cells also include cells in which no substantial Foxp3 expression is detected.

In the present invention, the above-mentioned cell population can be selected by using the following antibodies alone or in combination with each other.

Anti-CD4 antibody (OKT4, manufactured by eBioscience, Inc.), anti-CD25 antibody (M-A251, manufactured by BD Biosciences, Inc.), anti-human CCR4 antibody (1G1, manufactured by BD Biosciences, Inc.), anti-human CCR4 mouse monoclonal antibody (KM2160, Niwa et al, Cancer Res., 2004; 64: 2127-2133), anti-Foxp3 antibody (PCH101, manufactured by eBioscience, Inc.), and anti-IFN-y antibody (B27, manufactured by BD Biosciences, Inc.).

As one embodiment of the present invention, a therapeutic or preventive method and a therapeutic or preventive agent including decreasing HTLV-1-infected cells in the peripheral blood or CSF of a HAM patient by administering a low dose of an anti-human CCR4 antibody or an antibody fragment thereof to the HAM patient are exemplified.

In the present invention, the administering a low dose of an anti-human CCR4 antibody or an antibody fragment thereof refers to administering a small amount of an anti-human CCR4 antibody or an antibody fragment thereof at long intervals. The small amount of an anti-human CCR4 antibody or an antibody fragment thereof refers to an anti-human CCR4 antibody or an antibody fragment thereof in an amount as follows in a stepwise manner: preferably 1 mg/kg or less, 0.3 mg/kg or less, 0.1 mg/kg or less, 0.03 mg/kg or less, and 0.01 mg/kg or less, more preferably 0.3 mg/kg or less, and 0.1 mg/kg or less.

The administering an anti-human CCR4 antibody or an antibody fragment thereof at long intervals refers to administering an anti-human CCR4 antibody or an antibody fragment thereof at intervals as follows in a stepwise manner: preferably 4 weeks or more, 5 weeks or more, 6 weeks or more, 7 weeks or more, 8 weeks or more, 9 weeks or more, 10 weeks or more, 11 weeks or more, and 12 weeks or more, more preferably 8 weeks or more, 9 weeks or more, 10 weeks or more, 11 weeks or more, and 12 weeks or more, further more preferably 12 weeks or more.

In the present invention, an anti-human CCR4 antibody or an antibody fragment thereof is preferably administered at a dose of 1 mg/kg or less at administration intervals of 4 weeks or more, and most preferably administered at a dose of 0.3 mg/kg at administration intervals of 12 weeks.

As one embodiment of the present invention, a therapeutic or preventive method and a therapeutic or preventive agent including decreasing HTLV-1-infected cells in the peripheral blood or CSF of a HAM patient by inhibiting or removing target cells such as CD4+T cells, CCR4+ T cells, CD4+ CD25+ T cells, CD4+ CD25+ Foxp3 low T cells, CD4+ CD25+ CCR4+ T cells, CD4+ CD25+ CCR4+ Foxp3 low T cells, CD4+ CD25+ CCR4+ Foxp3 low IFN-$\gamma$+ T cells, or CD8+ CCR4+ T cells using an anti-human CCR4 antibody or an antibody fragment thereof are exemplified.

In the present invention, the decreasing HTLV-1-infected cells in the peripheral blood or CSF of a HAM patient refers to the following. In general, PBMCs of a healthy person hardly spontaneously proliferate without stimulation by an antibody, a cytokine, a chemical substance, or the like in vitro, however, PBMCs of a HAM patient spontaneously proliferate without any special stimulation. Therefore, in the present invention, the decreasing HTLV-1-infected cells in the peripheral blood or CSF of a HAM patient also includes decreasing the number of HTLV-1-infected cells as a result of inhibiting the spontaneous proliferation of PBMCs of the HAM patient using an anti-human CCR4 antibody or an antibody fragment thereof.

Further, as one embodiment of the present invention, a therapeutic or preventive method and a therapeutic or preventive agent including decreasing the number of HTLV-1-infected cells by cell proliferation inhibition specific to the HTLV-1-infected cells using an anti-human CCR4 antibody or an antibody fragment thereof, damage and removal or the like of the HTLV-1-infected cells using the effector activity of an anti-human CCR4 antibody or an antibody fragment thereof are also exemplified.

Further, as one embodiment of the present invention, a therapeutic or preventive method and a therapeutic or preventive agent including decreasing the amount of HTLV-1 proviral DNA in at least one of the peripheral blood and CSF of a HAM patient using a low dose of an anti-human CCR4 antibody or an antibody fragment thereof are also exemplified.

In the present invention, the decreasing the amount of HTLV-1 proviral DNA in the peripheral blood of a HAM patient refers to decreasing the amount of HTLV-1 proviral DNA contained in PBMCs of a HAM patient, and indicates a decrease in HTLV-1-infected cells per se in PBMCs, a decrease in new infection of cells in PBMCs (decrease in infection rate), or the like.

In the present invention, the decreasing the amount of HTLV-1 proviral DNA in CSF of a HAM patient indicates a decrease in HTLV-1-infected cells per se in CSF, a decrease in new infection of cells in CSF (decrease in infection rate), a decrease in migration of HTLV-1 -infected cells from the peripheral blood to CSF, or the like.

The amount of HTLV-1 proviral DNA can be measured based on a known method [Yamano et al, Blood, 2002: 99(1); 88-94]. That is, the copy number of HTLV-1 proviral DNA in PBMCs or CSF can be measured by amplifying a partial fragment of HTLV-1 pX gene using a specific primer and a cDNA derived from PBMCs of a HAM patient as a template.

As one embodiment of the present invention, a therapeutic method including decreasing the amount of at least one of neopterin and CXCL10 in CSF of a HAM patient by administering a low dose of an anti-human CCR4 antibody or an antibody fragment thereof is exemplified.

CXCL10 in CSF is considered to be associated with chronic inflammation in HAM, and further, the amount of neopterin or CXCL10 in CSF is known as a biomarker for inflammation in the spinal cord of a HAM patient. Therefore, as one embodiment of the present invention, a therapeutic or preventive method and a therapeutic or preventive agent, characterized by suppressing inflammation in the spinal cord of a HAM patient by administering a low dose of an anti-human CCR4 antibody or an antibody fragment thereof are exemplified.

The amount of neopterin and the amount of CXCL10 in CSF of a HAM patient can be measured by a known method [for example, a method described in Sato et al, PLOS Neglected Tropical Disease, 2013; 7(10): e2479]. That is, the amount of CXCL10 in CSF can be measured by flow cytometry using a cytometric bead array kit (manufactured by BD Biosciences, Inc.). Further, the amount of neopterin in CSF can be measured using high performance liquid chromatography (HPLC).

As one embodiment of the present invention, a therapeutic or preventive agent and a therapeutic or preventive method, characterized by decreasing the number of cells in CSF of a HAM patient by administering a low dose of an anti-human CCR4 antibody or an antibody fragment thereof are exemplified.

The number of cells in CSF of a HAM patient can be measured by a known method [for example, a method described in Sato et al, PLOS Neglected Tropical Diseases, 2013; 7(10): e2479]. That is, the number of cells in CSF can be measured using a cell counter plate such as a Fuchs-Rosenthal type.

The anti-human CCR4 antibody or the antibody fragment thereof used in the present invention may be any anti-human CCR4 antibody or antibody fragment thereof as long as it specifically binds to CCR4, however, preferably, an antibody or an antibody fragment thereof that specifically binds to an extracellular region of CCR4, an antibody or an antibody fragment thereof that inhibits binding of TARC/CCL17 or MDC/CCL22 to CCR4, an antibody having an effector activity, an antibody or an antibody fragment thereof that binds to an extracellular region of CCR4 and has an effector activity, an antibody or an antibody fragment thereof that binds to an extracellular region of CCR4 but does not bind to a platelet, an antibody or an antibody fragment thereof that binds to an extracellular region of CCR4, but does not bind to a platelet, and has an effector activity, etc. are exemplified.

Human CCR4 is a G protein coupled seven transmembrane receptor cloned as K5-5 from a human immature basophilic cell line KU-812, and has an amino acid sequence represented by SEQ ID NO: 9. The extracellular regions of CCR4 are amino acid sequences at positions 1-39, positions 99-111, positions 176-206, and positions 268-284 from the N-terminus of the amino acid sequence represented by SEQ ID NO: 9, and the intracellular regions are amino acid sequences at positions 68-77, positions 134-150, positions 227-242, and positions 309-360 from the N-terminus of the amino acid sequence represented by SEQ ID NO: 9 (UniProtKB/Swiss-Prot, ID: P51679).

Further, it is known that TARC (thymus and activation-regulated chemokine) produced from thymus cells (J. Biol. Chem., 271, 21514, 1996) and MDC (macrophage-derived chemokine) isolated from macrophages (J. Exp. Med., 185, 1595, 1997) also known as STCP-1 (stimulated T cell chemotactic protein-1) (J. Biol. Chem., 272, 25229, 1997) specifically bind to CCR4.

Therefore, as the anti-human CCR4 antibody or the antibody fragment thereof used in the present invention, preferably, an antibody or an antibody fragment thereof that binds to an epitope included in the extracellular regions composed of amino acid sequences at positions 1-39, positions 99-111, positions 176-206, and positions 268-284 from the N-terminus of the amino acid sequence represented by SEQ ID NO: 9, more preferably, an antibody or an antibody fragment thereof that binds to an epitope included in an amino acid sequence at positions 1-39 from the N-terminus of the amino acid sequence represented by SEQ ID NO: 9, and further more preferably, an antibody or an antibody fragment thereof that binds to an epitope included in an amino acid sequence at positions 2-29 from the N-terminus of the amino acid sequence represented by SEQ ID NO: 9, etc. are exemplified.

The anti-human CCR4 antibody used in the present invention may be any of a monoclonal antibody and a polyclonal antibody, but is preferably a monoclonal antibody that binds to a single epitope.

The monoclonal antibody may be any monoclonal antibody of a monoclonal antibody produced from a hybridoma and a recombinant antibody prepared by a genetic recombination technique.

In order to reduce immunogenicity in humans, a human chimeric antibody (hereinafter, also referred to as chimeric antibody), a humanized antibody [also referred to as human complementarity determining region (CDR)-grafted antibody], and a human antibody are preferably used.

The chimeric antibody is an antibody composed of a heavy chain variable region (hereinafter, abbreviated to VH) and a light chain variable region (hereinafter, abbreviated to VL) of a non-human animal antibody, and a heavy chain constant region (hereinafter, abbreviated to CH) and a light chain constant region (hereinafter, abbreviated to CL) of a human antibody. The type of animal for the variable region is not particularly limited as long as it is an animal capable of producing a hybridoma, such as a mouse, a rat, a hamster, a rabbit, or the like.

The human chimeric antibody can be prepared by obtaining cDNAs encoding VH and VL of a non-human animal antibody that specifically binds to human CCR4, inserting each of the cDNAs into an expression vector having genes encoding CH and CL of a human antibody, thereby constructing a human chimeric antibody expression vector, and then introducing the vector into animal cells so as to express the antibody.

The CH of the human chimeric antibody is not particularly limited as long as it is a human immunoglobulin (hereinafter, abbreviated to hIg), but is preferably CH of hIgG class. The CL of the human chimeric antibody is not particularly limited as long as it belongs to hIgG.

The humanized antibody is an antibody prepared by grafting of CDRs of VH and VL of a non-human animal antibody into appropriate sites of VH and VL of a human antibody. The human CDR-grafted antibody can be prepared by constructing cDNAs encoding V regions where CDRs of VH and VL of a non-human animal antibody that specifically binds to CCR4 are grafted into frameworks (hereinafter, abbreviated to FRs) of VH and VL of an arbitrary human antibody, inserting each of the cDNAs into an expression vector having DNAs encoding CH and CL of a human antibody, thereby constructing a humanized antibody expression vector, and then introducing the expression vector into animal cells so as to express the antibody. The amino acid sequences of the FRs of VH and VL of a human antibody are not particularly limited as long as they are amino acid sequences derived from a human antibody.

The CH of the humanized antibody is not particularly limited as long as it is hIg, but is preferably CH of hIgG class. The CL of the humanized antibody is not particularly limited as long as it belongs to hIg.

The anti-human CCR4 antibody fragment used in the present invention includes fragments of the above-mentioned respective antibodies. The type of the antibody fragment is not particularly limited, and for example, Fab, Fab', $F(ab')_2$, scFv, a diabody, dsFv, a CDR-containing peptide, etc. are exemplified.

The Fab is an antibody fragment having a molecular weight of about 50,000 and an antigen binding activity among fragments obtained by treating IgG with papain (protease). The Fab of the anti-human CCR4 antibody can be prepared by treating the anti-human CCR4 antibody with papain or by inserting a DNA encoding the Fab of the antibody into an expression vector, and then introducing the vector into a prokaryote or a eukaryote so as to express the Fab.

The $F(ab')_2$ is an antibody fragment having a molecular weight of about 100,000 and an antigen binding activity among fragments obtained by treating IgG with pepsin (protease). The $F(ab')_2$ of the anti-human CCR4 antibody can be prepared by treating the anti-human CCR4 antibody with pepsin or by binding Fab' (described below) through a thioether bond or a disulfide bond.

The F(ab') is an antibody fragment having a molecular weight of about 50,000 and an antigen binding activity, which is obtained by cutting a disulfide bond of the hinge region of the $F(ab')_2$. The Fab' of the anti-human CCR4 antibody can be prepared by treating the $F(ab')_2$ of the anti-human CCR4 antibody with dithiothreitol, or by inserting a DNA encoding the Fab' of the antibody into an expression vector, and then introducing the vector into a prokaryote or a eukaryote so as to express the F(ab').

The scFv is an antibody fragment having an antigen binding activity obtained by linking one VH and one VL using an appropriate peptide linker. The scFv of the anti-human CCR4 antibody can be prepared by obtaining cDNAs encoding VH and VL of the anti-human CCR4 antibody, constructing a DNA encoding scFv, inserting the DNA into an expression vector, and then introducing the expression vector into a prokaryote or a eukaryote so as to express the scFv.

The diabody is an antibody fragment obtained by dimerization of scFv, and has a divalent antigen binding activity. The diabody of the anti-human CCR4 antibody can be prepared by obtaining cDNAs encoding VH and VL of the anti-human CCR4 antibody, constructing a DNA encoding the diabody, inserting the DNA into an expression vector, and then introducing the expression vector into a prokaryote or eukaryote so as to express the diabody.

The dsFv is an antibody fragment obtained by binding polypeptides, in which one amino acid residue of each of VH and VL is substituted with a cysteine residue, via a disulfide bond between the cysteine residues. The dsFv of the anti-human CCR4 antibody can be prepared by obtaining cDNAs encoding VH and VL of the anti-human CCR4 antibody, constructing a DNA encoding the dsFv, inserting the DNA into an expression vector, and then introducing the expression vector into a prokaryote or a eukaryote so as to express the dsFv.

The CDR-containing peptide is a peptide containing at least one or more regions of CDRs of VH or VL. The peptide containing CDR of the anti-human CCR4 antibody can be prepared by constructing a DNA encoding CDRs of VH and VL of the anti-human CCR4 antibody, inserting the DNA into an expression vector, and then introducing the expression vector into a prokaryote or a eukaryote so as to express the peptide. Also, the peptide containing CDR of the anti-human CCR4 antibody can be prepared by a chemical synthesis method such as an Fmoc method (fluorenylmethyloxycarbonyl method) or a t-butyloxycarbonyl method.

In the present invention, the effector activity refers to an activity caused through the Fc region of an antibody, and for example, an antibody-dependent cellular cytotoxicity activity (ADCC activity), a complement-dependent cytotoxicity activity (CDC activity), antibody-dependent phagocytosis (ADP activity) by phagocytes such as macrophages or dendritic cells, etc. are exemplified.

As a method for controlling the effector activity, for example, a method for controlling the amount of fucose (also referred to as core fucose) which is bound through an α1-6 bond to N-acetylglucosamine (G1cNAc) present at a reducing end of a complex type N-linked sugar chain which is bound to asparagine (Asn) at position 297 of the EU index (Kabat et al, Sequence of Proteins of immunological interests, 5th edition, 1991) in the Fc region of an antibody (WO 2005/035586, WO 2002/31140, WO 00/61739), a method for controlling the effector activity by modifying amino acid residues in the Fc region of an antibody, etc. are exemplified.

The effector activity of the antibody can be increased or decreased by controlling the content of core fucose in the complex type N-linked sugar chain which is bound to Fc of the antibody. As a method for decreasing the content of fucose which is bound to a complex type N-linked sugar chain bound to Fc of the antibody, an antibody to which fucose is not bound can be obtained by expressing the antibody using CHO cells deficient in an α1,6-fucosyltransferase gene (FUT8).

The antibody to which fucose is not bound has a high ADCC activity. On the other hand, as a method for increasing the content of fucose which is bound to a complex type N-linked sugar chain bound to Fc of the antibody, an antibody to which fucose is bound can be obtained by expressing the antibody using a host cell into which an α1,6-fucosyltransferase gene is introduced. The antibody to which fucose is bound has a lower ADCC activity than the antibody to which fucose is not bound.

Further, by modifying an amino acid residue in the Fc region of the antibody, the ADCC activity or the CDC activity can be increased or decreased. By modifying an amino acid residue in the Fc region so as to increase or decrease the binding activity to FcγR, the ADCC activity can be controlled, and by modifying an amino acid residue in the Fc region so as to increase or decrease the binding activity of the complement, the CDC activity can be controlled.

For example, the CDC activity of the antibody can be increased by using the amino acid sequence of the Fc region described in the specification of US Patent Application Publication No. 2007/0148165. Further, the ADCC activity or the CDC activity can be increased or decreased by modifying an amino acid residue described in the specifications of US Patent No. 6,737,056, US Patent No. 7,297,775, and US Patent No. 7,317,091 and WO 2005/070963.

As the anti-human CCR4 antibody or the antibody fragment thereof used in the present invention, an anti-human CCR4 antibody or an antibody fragment thereof that binds to an epitope included in an amino acid sequence at positions 2-29 from the N-terminus of the amino acid sequence represented by SEQ ID NO: 9, an anti-human CCR4 antibody or an antibody fragment thereof that binds to the epitope and has an ADCC activity, an anti-human CCR4 antibody or an antibody fragment thereof that comprises H chain CDRs 1-3 comprising amino acid sequences represented by SEQ ID NOS: 1-3, respectively, and L chain CDRs 1-3 comprising amino acid sequences represented by SEQ ID NOS: 4-6, respectively, and an anti-human CCR4 antibody or an antibody fragment thereof that comprises VH comprising an amino acid sequence represented by SEQ ID NO: 7 and VL comprising an amino acid sequence represented by SEQ ID NO: 8 are exemplified. Further, an antibody, in which core fucose bound at position 297 of Fc of the above-mentioned antibody is reduced or deleted, is preferred. More specifically, an anti-human CCR4 humanized antibody (Poteligeo (registered trademark), general name: Mogamulizumab) is exemplified.

As one embodiment of the present invention, a combination therapy in which a treatment is performed by combining a low dose of an anti-human CCR4 antibody or an antibody fragment thereof and another therapeutic agent is also included.

In the combination therapy of the present invention, for example, a low dose of an anti-human CCR4 antibody or an antibody fragment thereof and an immunosuppressant can be used in combination. In the combination therapy of the present invention, an anti-human CCR4 antibody or an antibody fragment thereof and an agent to be used in combination therewith may be administered simultaneously or sequentially.

Examples of the immunosuppressant include adrenocorticosteroid drugs such as prednisolone, methylprednisolone, dexamethasone, and betamethasone, antimetabolites such as azathioprine, calcineurin inhibitors such as cyclosporine and tacrolimus (FK-506), Janus kinase (JAK) inhibitors such as tofacitinib and tasocitinib, CTLA4-Ig drugs prepared by fusion of cytolytic T lymphocyte associated antigen-4 (CTLA-4) with an antibody Fc region such as abatacept, and NF-κB inhibitors, which are drugs capable of suppressing excessive immune reactions of HAM. Further, derivatives of the drugs described above that act in the same manner on a molecule targeted by each drug can also be used.

In general, 10-60 mg of prednisolone is used for chronic inflammation symptoms of HAM patients. However, because long-term administration of prednisolone causes an adverse event such as obesity, diabetes, osteoporosis, glaucoma, or an infectious disease, it is necessary to control the using amount according to the inflammation symptoms of HAM patients.

The combination therapy of the present invention can exhibit a stronger antiinflammatory effect with a relatively low dose of an adrenocorticosteroid by using a low dose of an anti-human CCR4 antibody or an antibody fragment thereof. Therefore, the combination therapy of the present invention includes a therapeutic method using an anti-human CCR4 antibody or an antibody fragment thereof and a low dose of an adrenocorticosteroid in combination simultaneously or sequentially. Further, a method in which a low dose of an adrenocorticosteroid is used for a long period of time by using an anti-human CCR4 antibody or an antibody fragment thereof is also included. Further, a therapeutic method in which an anti-human CCR4 antibody or an antibody fragment thereof and a low dose of an adrenocorticosteroid are used in combination simultaneously or sequentially, characterized by reducing or preventing the onset of an adverse event accompanying the long-term use of an adrenocorticosteroid drug by the combination therapy of the present invention is also included. In the combination therapy of the present invention, an anti-human CCR4 antibody or an antibody fragment thereof and an adrenocorticosteroid may be administered simultaneously or sequentially.

In the present invention, the low dose of an adrenocorticosteroid is exemplified by, for example in terms of prednisolone, a daily dose of 1-10 mg, and hereinafter in a stepwise manner, preferably 9.5 mg, 9 mg, 8.5 mg, 8 mg, 7.5 mg, 7 mg, 6.5 mg, 6 mg, 5.5 mg, 5 mg, 4.5 mg, 4 mg, 3.5 mg, 3 mg, 2.5 mg, 2 mg, 1.5 mg, and 1 mg.

The therapeutic or preventive agent for HAM of the present invention may be any as long as it is a therapeutic or preventive agent characterized in that an anti-human CCR4 antibody or an antibody fragment thereof is contained as an active ingredient and is administered at a low dose, but is preferably provided as a pharmaceutical preparation produced by generally mixing with one or more pharmacologically acceptable carriers according to any method well known in the pharmaceutical technical field.

Preferably, an aseptic solution where it is dissolved in an aqueous carrier such as water, or an aqueous solution of sodium chloride, glycine, glucose, human albumin, or the like is used. Further, it is also possible to add a pharmacologically acceptable additive such as a buffer or a tonicity agent for making the preparation solution more similar to physiological conditions, for example, sodium acetate, sodium chloride, sodium lactate, potassium chloride, sodium citrate, or the like. Further, it can also be preserved by freeze-drying, and used by dissolving it in an appropriate solvent immediately before use.

As for the administration route of the therapeutic or preventive agent of the present invention, it is preferred to use the most effective route in the treatment, and oral administration or parenteral administration such as intraoral, tracheobronchial, intrarectal, subcutaneous, intramuscular, intrathecal, and intravenous administration can be exemplified, but intrathecal or intravenous administration is preferred.

Examples of the preparation suitable for the oral administration include an emulsion, a syrup, a capsule, a tablet, a powder, and a granule. For example, a liquid preparation such as an emulsion or a syrup can be produced using water, a saccharide such as sucrose, sorbitol or fructose, a glycol such as polyethylene glycol or propylene glycol, an oil such as sesame oil, olive oil, or soybean oil, a preservative such as an ester of p-hydroxybenzoic acid, a flavor such as a strawberry flavor or a peppermint flavor, or the like as an additive.

A capsule, a tablet, a powder, a granule, or the like can be produced using an excipient such as lactose, glucose, sucrose, or mannitol, a disintegrating agent such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, or the like, as an additive.

Examples of the preparation suitable for parenteral administration include an injection, a suppository, and a spray. For example, the injection is prepared using a carrier composed of a salt solution, a glucose solution, or a mixture thereof, or the like. The suppository is prepared using a carrier such as cacao butter, a hydrogenated fat, or a carboxylic acid. Further, the spray is prepared using a carrier which does not stimulate the antibody itself, and the mouth and the airway mucous membrane of a recipient, and disperses the antibody as fine particles so as to facilitate the absorption, or the like.

Specific examples of the carrier include lactose and glycerin. It is possible to formulate an aerosol, a dry powder, or the like according to the property of the antibody and the carrier to be used. Further, it is also possible to add a component exemplified as the additive in the oral preparation even in such a parenteral preparation.

Further, as the therapeutic or preventive agent for HAM of the present invention, a therapeutic agent for HAM containing a low dose of an anti-human CCR4 antibody or an antibody fragment thereof and a low dose of an adrenocorticosteroid, a therapeutic agent for HAM containing a low dose of an anti-human CCR4 antibody or an antibody fragment thereof and an adrenocorticosteroid, characterized by using a low dose of the adrenocorticosteroid in combination, a therapeutic agent for HAM containing a low dose of an anti-human CCR4 antibody or an antibody fragment thereof and an adrenocorticosteroid, characterized by using a low dose of the adrenocorticosteroid in combination simultaneously or sequentially, a therapeutic agent containing a low dose of an anti-human CCR4 antibody or an antibody fragment thereof and a low dose of an adrenocorticosteroid, characterized by using the low dose of the adrenocorticosteroid for a long period of time, and a therapeutic agent containing an anti-human CCR4 antibody or an antibody fragment thereof and a low dose of an adrenocorticosteroid, characterized by using the low dose of the adrenocorticosteroid simultaneously or sequentially for a long period of time are exemplified.

The therapeutic or preventive method for HAM and the therapeutic or preventive agent for HAM of the present invention can also be applied to an active treatment of an asymptomatic HTLV-1 carrier or an inactive HAM patient. By the active treatment of the AC, the treatment before the onset of chronic inflammation symptoms can be performed, and therefore, the occurrence of nerve damage or tissue damage can be suppressed.

Further, the active treatment of the inactive HAM patient can give the patient a recovery period of nerve damage or tissue damage that occurs during a HAM active period by suppressing chronic inflammatory response, and thus is important also in the improvement of symptoms and quality of life (QOL).

That is, the present invention also includes a method for reducing the risk of the onset of HAM by decreasing HTLV-1-infected cells in at least one of the peripheral blood and CSF of a patient, a method for reducing the risk of the onset of HAM by decreasing the amount of HTLV-1 proviral DNA in at least one of the peripheral blood and CSF of a patient, and a method for reducing the risk of the onset of HAM by suppressing the production of a cytokine derived from HTLV-1-infected cells in at least one of the peripheral blood and CSF of a patient by administrating a low dose of an anti-human CCR4 antibody or an antibody fragment thereof in a HTLV-1 carrier with a high risk of HAM, who is asymptomatic although the amount of an anti-HTLV-1 antibody, HTLV-1 proviral DNA, or the like is detected in the peripheral blood or CSF.

Further, the present invention also includes a preventive agent for HAM containing a low dose of an anti-human CCR4 antibody or an antibody fragment thereof, characterized by reducing the risk of the onset of HAM by decreasing HTLV-1-infected cells in the peripheral blood and CSF of a patient in an asymptomatic HTLV-1 carrier with a high risk of HAM.

The HTLV-1 carrier with a high risk of the onset of HAM can be distinguished by a diagnostic marker selected from the anti-HTLV-1 antibody titer, the amount of HTLV-1 proviral DNA, the amount of HTLV-1 Tax mRNA, the ratio of the amount of HTLV-1 Tax mRNA/the amount of HTLV-1 proviral DNA, and the number of CD4+ CD25+ T cells in the peripheral blood or CSF, the concentration of neopterin in CSF, the ratio of the amount of HTLV-1 proviral DNA in CSF/PBMC, soluble IL-2 receptor (sIL-2R), the concentration of CXCL10, HAM/ATL family medical history, and the like.

With respect to HAM patients, specifically, a HAM patient who is recognized to have at least one risk factor selected from a high amount level of HTLV-1 proviral DNA in the peripheral blood, a high concentration level of serum sIL-2R, a high concentration level of serum CXCL10, the presence of HAM/ATL family medical history, a high amount level of virus in CSF, an increased HTLV-1 antibody titer, a high concentration level of neopterin, and a high concentration level of CXCL10 can be a subject of the active treatment. The high level of each diagnostic marker means a relatively high level among the HAM patients.

Further, with respect to ACs, an AC who is recognized to have at least one risk factor selected from a high amount level of HTLV-1 proviral DNA, a high concentration level of serum sIL-2R, a high concentration level of serum CXCL10, and the presence of HAM/ATL family medical history can be a high-risk AC.

On the other hand, an AC having a low amount level of HTLV-1 proviral DNA, a low concentration level of serum sIL-2R, a low concentration level of serum CXCL10, no HAM/ATL family medical history, or the like can be a low-risk AC. The high level of each diagnostic marker means a relatively high level among the ACs, and also includes a case where the level is higher than the HAM diagnostic level.

Further, the present invention also includes a method for reducing the severity of HAM by decreasing HTLV-1 -infected cells in at least one of the peripheral blood and CSF of a HAM patient, a method for reducing the severity of HAM by decreasing the number of cells in CSF of a HAM patient, a method for reducing the severity of HAM by decreasing the amount of HTLV-1 proviral DNA in at least one of the peripheral blood and CSF of a HAM patient, and a method for reducing the severity of HAM by suppressing the production of at least one of neopterin and CXCL10 in CSF of a HAM patient by administrating a low dose of an anti-human CCR4 antibody or an antibody fragment thereof in an inactive HAM patient whose motor disability degree is not high.

Further, the present invention also includes a method for decreasing the amount of HTLV-1 proviral DNA in at least one of the peripheral blood and CSF of a HAM patient using a low dose of an anti-human CCR4 antibody or an antibody fragment thereof, a method for decreasing the number of cells in CSF of a HAM patient using a low dose of an anti-human CCR4 antibody or an antibody fragment thereof, and a method for decreasing the amount of at least one of neopterin and CXCL10 in CSF of a HAM patient.

The therapeutic or preventive method for HAM and the therapeutic or preventive agent for HAM of the present invention can also be applied to prevention of the onset of ATL in a HAM patient or an AC. A method for preventing the onset of ATL by decreasing CADM1$^+$ CD7$^{-/dim}$CD4$^+$ cells [Kobayashi et al, Clinical Cancer Research, 2014: 20(11); 2851-61] in the peripheral blood of a HAM patient or an AC using a low dose of an anti-human CCR4 antibody or an antibody fragment thereof is also included in the present invention.

EXAMPLES

Hereinafter, the present invention will be described in more detail with reference to Examples, but the invention is not limited to these Examples.

Clinical Trial Using Anti-Human CCR4 Antibody Mogamulizumab Targeting HAM Patients

A clinical trial was performed as an open label phase I-2a trial targeting HAM patients using an anti-human CCR4 humanized antibody mogamulizumab.

[Patient Selection Criteria]

Patients selected according to the following criteria were enrolled in the clinical trial.

<Eligibility Criteria>

Patients who were diagnosed with HAM according to HAM/TSP diagnostic guidelines (Osame M. Review of WHO Kagoshima Meeting and Diagnostic Guidelines for HAM/TSP. In: Blattner WA, ed. Human Retrovirology: Htlv. New York: Raven Press, Ltd.; 1990: 191-7), and also meet the following conditions

(1) Patients who were confirmed to be positive for an anti-human T cell leukemia virus type-1 (HTLV-1) antibody in the serum and CSF
(2) Patients who developed HAM one year ago or earlier
(3) Patients who are undergoing steroid maintenance therapy and also fall under the following conditions:

- Patients who are undergoing continuous administration at a dose of 10 mg/day or less in terms of prednisolone for 3 months or more
- The concentration of neopterin in CSF is not improved to 5 pmol/mL or less
- Patients in whom a fluctuation in the concentration of neopterin in CSF immediately before enrollment was within a range of 60% of the concentration of neopterin in CSF at 3 months or more before enrollment (provided that the concentrations of neopterin to be compared are measured during steroid maintenance therapy) However, the following patients are excluded.
  Patients who received steroid pulse therapy within 3 months before enrollment
  Patients whose steroid oral dose was changed within 3 months before enrollment

(4) Patients whose age was 20 or above at the time when informed consent is obtained
(5) Patients in whom HTLV-1 proviral load in the peripheral blood can be detected
(6) Patients whose Osame's motor disability score (OMDS) grade did not change for 3 months or more before enrollment
(7) Patients who can walk 10 m or more regardless of whether a walking aid is used or not at the time when informed consent is obtained
(8) Patients who can be hospitalized until one week after completion of administration of mogamulizumab
(9) Patients in whom primary organ functions are maintained (Patients whose latest test data within 28 days before enrollment meet the following criteria)

- neutrophil count: 1,500/mm$^3$ or more
- platelet count: 100,000/mm$^3$ or more
- hemoglobin: 9.0 g/dL or more
- AST (GOT): 1.5 times or less of the upper limit of facility reference value
- ALT (GPT): 1.5 times or less of the upper limit of facility reference value
- total bilirubin: 1.5 times or less of the upper limit of facility reference value
- serum creatinine: 1.5 times or less of the upper limit of facility reference value
- electrocardiogram: abnormal finding requiring treatment is not observed
- left ventricular ejection fraction (LVEF) by echocardiography: 50% or more
- blood oxygen saturation (SpO$_2$): 90% or more

(10) Patients whose written informed consent for participation in the present clinical trial was obtained from the patients by own free will.

<Exclusion Criteria>

In view of protection of test subjects and a problem in terms of evaluation of an investigational drug, patients who fall under any of the following criteria are excluded from the present clinical trial.

(1) Patients with a past history of acute hepatitis, chronic hepatitis, or hepatic cirrhosis
(2) Patients with a past history of tuberculosis or with current active tuberculosis

(3) Patients who developed myocardial infarction within 12 months before enrollment

(4) Patients who developed allergic symptoms by administration of an antibody preparation in the past

(5) Patients who received administration of an immunosuppressant interferon-$\alpha$ within 6 months before enrollment

(6) Patients who underwent live or attenuated/inactivated vaccination within 4 weeks before enrollment, or who plan to be vaccinated during the course of the clinical trial

(7) Patients with a serious complication (heart failure, lung disease, renal failure, liver failure, hard-to-control diabetes, or the like)

(8) Other patients with a disease whose symptoms could be exacerbated by the administration of mogamulizumab (bedsore, an infectious disease, or an autoimmune disease)

(9) Patients with a past history of cancer with a complication

However, patients who underwent radical resection of solid tumors and have not recurred for at least 5 years before enrollment can be enrolled. Further, with respect to basal cell carcinoma of the skin, squamous cell carcinoma (except for malignant melanoma), non-invasive cervix carcinoma, carcinoma in situ in the gastrointestinal tract, and carcinoma in situ in the corpus uteri, patients who were determined to be completely cured can be enrolled even if recurrence occurred within 5 years.

(10) Patients with a complication of ATL

(11) Patients who are pregnant, breastfeeding, or may be pregnant or patients who want to bear a child

(12) Patients who received administration of a vitamin preparation (Alinamin, vitamin C, or the like) or the following supplement (fucoidan, catechin, or pentosan polysulfate) within 2 weeks before enrollment

(13) Patients who received administration of another investigational drug within 4 months before informed consent for participation in the clinical trial was obtained

(14) Patients with a complication of spinal cord compression lesion such as a cervical disease, intervertebral disk displacement, or ossification of the ligamentum flavum

(15) Patients with mental disorder, seizure, or dementia

(16) Patients who are positive for HBs antigen, or HBc antibody or HBV-DNA (real-time PCR method)

(17) Patients who are positive for HCV antibody

(18) Patients who are positive for HIV antibody

(19) Other patients determined to be unqualified to participate in the present clinical trial by a principal investigator or a sub investigator

Twenty-one patients (female: 17, male: 4) were enrolled in the phase I trial according to the above-mentioned criteria, and the median age was 64 years (range: from 36 to 73 years), the median disease duration was 9.2 years (range: from 2.2 to 35.1 years), and the median OMDS score was 5 (range: from 2 to 6). The median dose of oral prednisolone administered to the patients at the time of enrollment was 5 mg/day (range: from 2.5 to 10 mg/day), and the median treatment period with oral prednisolone before start of the present trial was 3.5 years (range: from 0.5 to 7.8 years).

[Research Treatment]

The phase I trial was performed as a dose-escalation study. On the administration day (hereinafter also referred to as Day 1), mogamulizumab was intravenously administered to the patients, and observation was performed until Day 85 (which is the 85th day based on the administration day, hereinafter, a day is expressed in the same manner). The determination of the maximum tolerated dosage (MTD) was performed at 5 dose levels from 0.003 to 0.3 mg/kg according to the 3+3 design. A relationship between each dose level and the dose of mogamulizumab is shown in Table 1.

[Table 1]

Table 1

| Dose level | Dose of mogamulizumab (mg/kg) |
|---|---|
| 1 | 0.003 |
| 2 | 0.01 |
| 3 | 0.03 |
| 4 | 0.1 |

(continued)

| Dose level | Dose of mogamulizumab (mg/kg) |
|---|---|
| 5 | 0.3 |

The target for dose limiting toxicity (hereinafter abbreviated to DLT) was determined to be hematotoxicity at grade 3 or higher (in the case of leukopenia, neutropenia, and lymphopenia, only grade 4 or higher) or non-hematotoxicity at grade 3 or higher (in the case of infusion reaction/cytokine release syndrome, only grade 4) manifested not later than 1 week after administering mogamulizumab. The number of cases to be investigated at each dose level was set to 3 (6 at the maximum). Enrollment of patients was performed from the dose level 1, and it was determined that the dose level is shifted higher in a stepwise manner according to the following rule.
(1) When the number of cases where a DLT event occurred was 0 in 3 cases, the dose level is shifted higher by one level.
(2) When the number of cases where a DLT event occurred was 1 or 2 in 3 cases, three cases are added to the same dose level so that the number of cases is 6.

(2-1) When the number of cases where a DLT event occurred was 1 or 2 in 6 cases, the dose level is shifted higher by one level.
(2-2) When the number of cases where a DLT event occurred was 3 or more in 6 cases, the dose level was determined to be MTD.

(3) When the number of cases where a DLT event occurred was 3 in 3 cases, the dose level was determined to be MTD.

When MTD was determined, a dose level that is lower by one level than MTD was determined to be the maximum dose. Further, when the number of cases where a DLT event occurred was 2 or less in 3 cases even at the dose level 5, the dose level 5 was determined to be the maximum dose. It was determined that 3 cases are added as the investigation cases at the dose level determined as the maximum dose.
On day 85, selection of patients who proceed to the phase IIa was performed. The initial administration in the phase IIa was performed at the same dose level as that in the phase I for each patient. Thereafter, administration was performed at a dose of 0.003, 0.01, or 0.03 mg/kg at intervals of 2 months (1 month corresponds to 28 days or 4 weeks) or at a dose of 0.1 or 0.3 mg/kg at intervals of 3 months. However, when the patients did not meet the following re-administration criteria, the administration was postponed.

<Re-Administration Criteria>

The patients who meet all the following criteria received re-administration as those who meet the re-administration criteria.

(1) Patients in whom an HTLV-1 provirus was detected in the peripheral blood in the test immediately before the administration day (in the case of the initial time, at the time of enrollment)
(2) Patients who were negative for an anti-mogamulizumab neutralizing antibody in the plasma
(3) Patients whose blood test values on the day before administration or the administration day meet the following conditions:

(i) neutrophil count: 1,500/mm$^3$ or more
(ii) platelet count: 100,000/mm$^3$ or more
(iii) hemoglobin: 9.0 g/dL or more
(iv) AST (GOT): 1.5 times or less of the upper limit of facility reference value
(v) ALT (GPT): 1.5 times or less of the upper limit of facility reference value
(vi) total bilirubin: 1.5 times or less of the upper limit of facility reference value
(vii) serum creatinine: 1.5 times or less of the upper limit of facility reference value

(4) Patients determined to have no problem in safety in administration by a principal investigator and a sub investigator

When the proviral level after administration did not decrease to 40% or less of the base line value on the day before administration (Day 0) in the phase I, administration was performed at a dose level shifted higher by one level. Incidentally, a steroid drug having been orally administered before participation in the clinical trial was used in combination without changing the dose during the trial period. In addition, before each administration of mogamulizumab, an antihistamine (30-50 mg of diphenhydramine or 2 mg of d-chlorpheniramine maleate) and an antipyretic analgesic (300-500 mg of acetaminophen) were orally administered.

The timing and the dose level at which administration was actually performed based on the above-mentioned trial regimen are shown in Table 2 for each patient.

[Table 2]

Table 2

| Patient | Phase 1 | Phase IIa | | | | | |
|---|---|---|---|---|---|---|---|
| | | 0 month | 1 month | 2 months | 3 months | 4 months | 5 months |
| 1 | Lv1 | Lv1 | | Lv2 | | Lv2 | |
| 2 | Lv1 | Lv1 | | Lv2 | | Lv3 | |
| 3 | Lv1 | Lv1 | nAb (+) | nAb (+) | nAb (+) | nAb (+) | nAb (+) |
| 4 | Lv2 | Lv2 | | Lv3 | | Lv4 | |
| 5 | Lv2 | Lv2 | | Lv2 | | Lv2 | |
| 6 | Lv2 | Lv2 | | Lv3 | | Lv3 | |
| 7 | Lv3 | Lv3 | | | Lv3 | | Lv3 |
| 8 | Lv3 | Lv3 | | | Lv3 | | Lv3 |
| 9 | Lv3 | Clinical trial was stooped due to DLT event on Day 15 in phase I | | | | | |
| 10 | Lv3 | Lv3 | | Lv4 | | | Lv4 |
| 11 | Lv3 | Lv3 | | Lv3 | | Lv3 | |
| 12 | Lv3 | Lv3 | | Lv3 | | Lv3 | |
| 13 | Lv4 | Lv4 | | nAb (+) | nAb (+) | nAb (+) | nAb (+) |
| 14 | Lv4 | Lv4 | | | Lv4 | | |
| 15 | Lv4 | Clinical trial was stopped before administration in phase IIa | | | | | |
| 16 | Lv5 | Lv5 | Administration was postponed due to rash or delay in nAb trial results | | | | |
| 17 | Lv5 | Lv5 | | | Lv5 | | |
| 18 | Lv5 | Lv5 | | | Lv5 | | |
| 19 | Lv5 | Lv5 | | | | Lv5 | |
| 20 | Lv5 | Lv5 | | | Lv5 | | |
| 21 | Lv5 | Lv5 | | | Lv5 | | |
| Lv: dose level; Lv1: 0.003 mg/kg, Lv2: 0.01 mg/kg, Lv3: 0.03 mg/kg, Lv4: 0.1 mg/kg, Lv5: 0.3 mg/kg, nAb: neutralizing antibody | | | | | | | |

[Evaluation of Drug Efficacy]

I. Evaluation for PBMCs

(1) Collection of PBMCs

The collection of peripheral blood was performed at the time of screening, on the day before administration of mogamulizumab (Day 0), and on Days 2, 7, 15, 29, 57, and 85 in the phase I, and every 4 weeks during the phase IIa period. In a blood collection tube containing disodium ethylenediaminetetraacetate (EDTA-2Na), blood was collected from a patient, and thereafter, mixing by inversion was promptly performed, and the resulting mixture was stored at room temperature until a specimen was recovered. PBMCs were isolated from the peripheral blood by a Ficoll density gradient centrifugation method and cryopreserved until they were used for analysis.

(2) Analysis of CCR4+ Cells

By using a fluorescently labeled anti-CCR4 antibody 1G1 (BD Biosciences, Inc.), each antibody at a saturation concentration was reacted with PBMCs at 4°C for 20 minutes in the dark. Thereafter, PBMCs were washed twice and then analyzed using FACS Calibur (BD Biosciences, Inc.).

Changes in the ratio of CCR4-positive cells in PBMCs based on those on Day 0 are shown in Fig. 1. As shown in Fig. 1, the CCR4-positive cells in PBMCs were promptly decreased by single administration of mogamulizumab at any dose level.

(3) Evaluation of Amount of Proviral DNA in PBMCs

The amount of proviral DNA in PBMCs was evaluated according to the method described in Yamano et al, Blood, 2002: 99(1); 88-94 as follows.

After PBMCs were suspended in a buffer containing 50 mM Tris-HCl (pH 8.0), 20 mM EDTA, 0.1 M NaCl, and 1% SDS (hereinafter referred to as lysis buffer), 150 $\mu$g/mL of proteinase K (manufactured by Wako Pure Chemical Industries, Ltd.) was added thereto, followed by shaking overnight at 55°C, and a genomic DNA of PBMCs of a HAM patient was extracted using phenol/chloroform.

By using the extracted genomic DNA as a template, and also using TaqMan probes for an HTLV-1 pX region and human $\beta$-actin, and a primer set of each gene, real time-polymerase chain reaction (hereinafter abbreviated to PCR) was performed.

Calibration curves were created by simultaneously performing PCR using a genomic DNA derived from an HTLV-1-infected rat TARL2 cell line integrated with an HTLV-1 pX region at 1 copy/cell as a standard specimen of HTLV-1 pX, and a genomic DNA derived from PBMCs of a healthy subject as a standard specimen of $\beta$-actin. The copy numbers of pX and $\beta$-actin in each specimen were calculated from the created calibration curves, and the amount of HTLV-1 proviral DNA per 100 PBMC cells was determined according to the following formula. The results are shown in Fig. 2 and Fig. 3.

$$\text{Amount of HTLV-1 proviral DNA per 100 PBMC cells} = \text{(copy number of HTLV-1 (pX)) / (copy number of } \beta\text{-actin/2)} \times 100$$

As shown in Fig. 2, the amount of proviral DNA in PBMCs was promptly decreased by single administration of mogamulizumab in the phase I trial at any dose level. Further, as shown in Fig. 3, a decrease in the amount of provirus in PBMCs was maintained by repetitive administration of mogamulizumab in the phase IIa trial.

The amount of HTLV-1 provirus in the peripheral blood is known to be correlated with the long-term prognosis of HAM (Olind et al, Arch. Neurol. 2006; 63: 1560-1566). Therefore, it was demonstrated that by administering a low dose of an anti-human CCR4 antibody or an antibody fragment thereof, the amount of HTLV-1 proviral DNA in the peripheral blood is decreased, and HAM can be cured.

II. Evaluation for CSF

(1) Collection of CSF

The collection of CSF was performed at the time of screening, on the day before administration of mogamulizumab (Day 0), and on Days 2, 7, 15, 29, 57, and 85 in the phase I, and at the time of initial administration and each re-administration thereafter in the phase IIa.

(2) Measurement of Number of Cells in CSF

The number of cells in CSF was counted using a Fuchs-Rosental type cell counter plate according to the attached instruction manual. The results are shown in Fig. 4.

As shown in Fig. 4, the number of cells in CSF was promptly decreased by single administration of mogamulizumab in the phase I trial at any dose level. Not less than 90% of the cells in CSF express CXCR3 that is a receptor for CXCL10, and are considered to be associated with an inflammation loop (Yamano et al, Clinical and Experimental Neuroimmunology, 2015: 6; 395-401).

Therefore, from the above results, it was demonstrated that by administering a low dose of an anti-human CCR4 antibody or an antibody fragment thereof, chronic inflammation in HAM patients can be suppressed.

(3) Measurement of Amount of HTLV-1 proviral DNA in CSF

The amount of HTLV-1 proviral DNA in CSF was measured according to the method described in I-(3) using cells obtained by centrifugation of CSF collected from a patient at 1,000 g for 10 minutes. The amount of HTLV-1 proviral DNA per 1 mL of CSF was calculated according to the following formula. The results are shown in Fig. 5 and Fig. 6.

$$\text{Amount of HTLV-1 proviral DNA per 1 mL of CSF (copy number/mL)} = \text{(copy number of pX)} / \text{(copy number of } \beta\text{-actin/2)} \times \text{(number of cells in 1 } \mu\text{L of CSF)} \times 1000$$

As shown in Fig. 5 and Fig. 6, the amount of HTLV-1 proviral DNA in CSF was decreased by single administration of mogamulizumab in the phase I trial and by repetitive administration of mogamulizumab in the phase IIa trial.

(4) Measurement of Concentrations of CXCL10 and Neopterin in CSF

In the measurement of the concentrations of CXCL10 and neopterin in CSF, the serum obtained by centrifugation of CSF collected from a patient at 1,000 g for 10 minutes were used. The measurement of the concentration of CXCL10 in CSF was performed using a cytometric bead array (BD Biosciences, Inc.) according to the instruction manual. Further, the concentration of neopterin in CSF was measured using high performance liquid chromatography (HPLC). The results are shown in Fig. 7 to Fig. 10.

As shown in Fig. 7 to Fig. 10, the concentrations of CXCL10 and neopterin in CSF were decreased by single administration of mogamulizumab in the phase I trial and by repetitive administration of mogamulizumab in the phase IIa trial.

CXCL10 in CSF is considered to be associated with chronic inflammation in HAM, and further, the concentrations of neopterin and CXCL10 in CSF are biomarkers for inflammation in the spinal cord of a HAM patient and are known to be strongly correlated with the rate of progression of the disease [Sato et al, PLOS Neglected Tropical Diseases, 2013: 7(10); e2479].

Accordingly, from the above results, it was demonstrated that by administering a low dose of an anti-human CCR4 antibody or an antibody fragment thereof, chronic inflammation in HAM patients can be suppressed.

III. Evaluation of Motor Function

Evaluation of Modified Ashworth Scale (MAS) and Osame's Motor Disability Score (OMDS)

The evaluation of MAS and OMDS was performed at the time of screening and on Days 0, 7, 15, 29, 57, and 85 in the phase I, and every 4 weeks and on the administration day of mogamulizumab during the phase IIa period.

MAS was scored by determining the conditions of patients according to the following Table 3. The results are shown in Fig. 11 and Fig. 12.

[Table 3]

Table 3

| Grade | Evaluation index |
|---|---|
| 0 | No increase in muscle tone |
| 1 | With slight increase in muscle tone, manifested by a catch and release, or by minimal resistance at the end of the range of motion when the affected part is moved in flexion or extension |

(continued)

| Grade | Evaluation index |
|---|---|
| 1+ | With slight increase in muscle tone, manifested by an apparent catch and minimal resistance in one-half or less of the range of motion |
| 2 | Increase in muscle tone is observed through most of the range of motion, but the affected part can be easily moved |
| 3 | With considerable increase in muscle tone, passive movement is difficult |
| 4 | Muscle is rigid, cannot perform flexion or extension |

As shown in Fig. 11, by single administration of mogamulizumab in the phase I trial, there were no patients at grade 2 or higher on Day 7 regardless of the dose level. Further, also on Day 85, the ratio of patients at grade 2 or higher was maintained at 10%. Further, as shown in Fig. 12, by repetitive administration of mogamulizumab in the phase IIa trial, the ratio of patients at grade 2 or higher was 10% or less after 3 months.

Accordingly, from the above results, it was demonstrated that by administering a low dose of an anti-human CCR4 antibody or an antibody fragment thereof, motor disorder due to spasm is improved, and HAM can be cured.

OMDS was scored by determining the conditions of patients according to the following Table 4. The results are shown in Fig. 13 and Fig. 14.

[Table 4]

Table 4

| Grade | Evaluation index |
|---|---|
| 0 | No walking or running abnormalities are observed |
| 1 | Walking speed is slow |
| 2 | Abnormal walking (stumbling, stiffness in knee) |
| 3 | Cannot run |
| 4 | Needs handrail to climb and descend stairs |
| 5 | Needs unilateral support to walk |
| 6 | Cannot walk with unilateral support, but can walk 10 m or more with bilateral support |
| 7 | Can walk 5 m or more and less than 10 m with bilateral support |
| 8 | Can walk less than 5 m with bilateral support |
| 9 | Cannot walk with bilateral support, but can crawl |
| 10 | Cannot crawl, but can move using arms |
| 11 | Cannot move around by oneself, but can turn over in bed |
| 12 | Cannot turn over in bed |
| 13 | Cannot even move toes |

As shown in Fig. 13, the ratio of patients at grade 5 or higher was 70% before start of the trial, however, by single administration of mogamulizumab in the phase I trial, the ratio of patients at grade 5 or higher was decreased to 60% or less on Day 7 to Day 15, and decreased to about 50% on Day 29. Further, patients at grade 2 were increased compared to those before administration of the antibody. In addition, as shown in Fig. 14, by repetitive administration of mogamulizumab in the phase IIa trial, a tendency of improving OMDS was maintained, and 10.5% of patients were confirmed to be further improved to grade 1.

Accordingly, it was demonstrated that by administering a low dose of an anti-human CCR4 antibody or an antibody fragment thereof, motor disorder is improved, and HAM can be cured.

To summarize the above results, it was demonstrated that by administering a low dose of an anti-human CCR4 antibody or an antibody fragment thereof, the amount of virus in PBMCs of a HAM patient is reduced and spinal inflammation is suppressed, and as a result, motor disorder is improved, and HAM can be cured.

IV. Results of Evaluation of Patients who Received Administration of Mogamulizumab at Dose Level 5

The results of evaluation of four patients (Nos. 17, 18, 20, and 21) to whom the anti-CCR4 antibody at dose level 5 was administered at intervals of 3 months (12 weeks) in the 19 patients who participated in the phase IIa trial are shown in Fig. 15(A) to Fig. 15(E).

As shown in Fig. 15(A) to Fig. 15(E), in these patients, although there is an individual difference in the effect, the amount of HTLV-1 proviral DNA in PBMCs and the concentrations of CXCL10 and neopterin in CSF were decreased, and further, maintenance or improvement of MAS and OMDS was observed.

Accordingly, it was demonstrated that by administering an anti-CCR4 antibody or an antibody fragment thereof at 0.3 mg/kg at intervals of 3 months (12 weeks), HAM can be cured.

V. Safety

Adverse events observed in the present trial are shown in Table 5.

[Table 5]

Table 5

| Adverse event | All grades | Grade 1 | Grade 2 | Grade 3 |
|---|---|---|---|---|
| | Number of patients (%) | | | |
| All adverse events | 19(90.5) | 4 (19.0) | 14 (66.7) | 1 (4.8) |
| Non-hematological adverse events | | | | |
| rash | 7 (33.3) | 4 (19.0) | 3 (14.3) | 0 (0.0) |
| hypoalbuminemia | 5 (23.8) | 5 (23.8) | 0 (0.0) | 0 (0.0) |
| hypoproteinemia | 4 (19.0) | 4 (19.0) | 0 (0.0) | 0 (0.0) |
| maculopapular rash | 4 (19.0) | 0 (0.0) | 4 (19.0) | 0 (0.0) |
| eczema | 3 (14.3) | 3 (14.3) | 0 (0.0) | 0 (0.0) |
| pruritus | 3 (14.3) | 3 (14.3) | 0 (0.0) | 0 (0.0) |
| increase in AST | 2 (9.5) | 1 (4.8) | 0 (0.0) | 1 (4.8) |
| stomatitis | 2 (9.5) | 1 (4.8) | 1 (4.8) | 0 (0.0) |
| fever | 2 (9.5) | 2 (9.5) | 0 (0.0) | 0 (0.0) |
| herpes labialis | 1 (4.8) | 1 (4.8) | 0 (0.0) | 0 (0.0) |
| herpes zoster | 1 (4.8) | 0 (0.0) | 1 (4.8) | 0 (0.0) |
| liver dysfunction | 1 (4.8) | 0 (0.0) | 1 (4.8) | 0 (0.0) |
| hypertension | 1 (4.8) | 0 (0.0) | 1 (4.8) | 0 (0.0) |
| infusion reaction | 1 (4.8) | 1 (4.8) | 0 (0.0) | 0 (0.0) |
| headache | 1 (4.8) | 1 (4.8) | 0 (0.0) | 0 (0.0) |
| proteinuria | 1 (4.8) | 1 (4.8) | 0 (0.0) | 0 (0.0) |
| hyperuricemia | 1 (4.8) | 1 (4.8) | 0 (0.0) | 0 (0.0) |
| hypokalemia | 1 (4.8) | 1 (4.8) | 0 (0.0) | 0 (0.0) |
| hypocalcemia | 1 (4.8) | 1 (4.8) | 0 (0.0) | 0 (0.0) |

(continued)

| Non-hematological adverse events | | | | |
|---|---|---|---|---|
| increase in GGT | 1 (4.8) | 0 (0.0) | 1 (4.8) | 0 (0.0) |
| increase in LDH | 1 (4.8) | 1 (4.8) | 0 (0.0) | 0 (0.0) |
| increase in ALT | 1 (4.8) | 0 (0.0) | 1 (4.8) | 0 (0.0) |
| increase in ALP | 1 (4.8) | 1 (4.8) | 0 (0.0) | 0 (0.0) |
| Hematological adverse events | | | | |
| lymphopenia | 7 (33.3) | 0 (0.0) | 7 (33.3) | 0 (0.0) |
| leukopenia | 7 33.3 | 4 (19.0) | 3 (14.3) | 0 (0.0) |
| eosinophilia | 1 (4.8) | 1 (4.8) | 0 (0.0) | 0 (0.0) |
| neutropenia | 1 (4.8) | 0 (0.0) | 1 (4.8) | 0 (0.0) |
| AST: aspartate aminotransferase, GGT: γ-glutamyltransferase, LDH: lactate dehydrogenase, ALT: alanine aminotransferase, ALP: alkaline phosphatase | | | | |

As shown in Table 5, mogamulizumab showed a favorable safety profile. It is known that when 1.0 mg/kg mogamulizumab was administered to ATL patients every week, an infusion reaction occurred in about 90% patients, and also serious skin rash at grade 3 or higher occurred at high frequency [Ishida et al, J. Clin. Oncol., 2012: 30(8); 837-842]. On the other hand, in the present trial, a mild infusion reaction at grade 1 was merely observed transiently only in one case. Further, skin rash at grade 3 or higher was not observed.

Accordingly, it was demonstrated that by administering a low dose of an anti-human CCR4 antibody or an antibody fragment thereof, an effective and highly safe treatment of HAM can be achieved.

SEQUENCE LISTING FREE TEXT

SEQ ID NO: 1: description of artificial sequence: amino acid sequence of H chain CDR1 of anti-human CCR4 antibody
SEQ ID NO: 2: description of artificial sequence; amino acid sequence of H chain CDR2 of anti-human CCR4 antibody
SEQ ID NO: 3: description of artificial sequence; amino acid sequence of H chain CDR3 of anti-human CCR4 antibody
SEQ ID NO: 4: description of artificial sequence; amino acid sequence of L chain CDR1 of anti-human CCR4 antibody
SEQ ID NO: 5: description of artificial sequence; amino acid sequence of L chain CDR2 of anti-human CCR4 antibody
SEQ ID NO: 6: description of artificial sequence; amino acid sequence of L chain CDR3 of anti-human CCR4 antibody
SEQ ID NO: 7: description of artificial sequence; amino acid sequence of H chain variable region of anti-human CCR4 antibody
SEQ ID NO: 8: description of artificial sequence; amino acid sequence of L chain variable region of anti-human CCR4 antibody

Although the present invention has been described in detail with reference to specific embodiments, it is apparent to those skilled in the art that various changes and modifications can be made without departing from the spirit and scope of the present invention. Incidentally, the present application is based on Japanese patent application (Japanese Patent Application No. 2017-039506) filed on March 2, 2017, the entire content of which is incorporated herein by reference.

## Claims

1. A preventive or therapeutic agent for human T cell leukemia virus type-1 (hereinafter abbreviated to HTLV-1) associated myelopathy (HTLV-1 associated myelopathy, hereinafter abbreviated to HAM), comprising:

   an anti-human CC-chemokine receptor 4 (CCR4) antibody or an antibody fragment thereof as an active ingredient,
   wherein the antibody or the antibody fragment thereof is administered at a low dose.

2. The preventive or therapeutic agent for HAM according to claim 1,
   wherein the anti-human CCR4 antibody or the antibody fragment thereof is administered at a dose of 1 mg/kg or less at administration intervals of 4 weeks or more.

3. The preventive or therapeutic agent for HAM according to claim 1 or 2,
   wherein the anti-human CCR4 antibody or the antibody fragment thereof is administered at a dose of 0.3 mg/kg at administration intervals of 12 weeks.

4. The preventive or therapeutic agent for HAM according to any one of claims 1 to 3,
   wherein the anti-human CCR4 antibody or the antibody fragment thereof is an anti-human CCR4 antibody or an antibody fragment thereof comprising an antibody heavy chain variable region (hereinafter, abbreviated to VH) comprising complementarily determining regions (hereinafter, abbreviated to CDRs) 1, 2, and 3 comprising amino acid sequences represented by SEQ ID NOS: 1, 2, and 3, respectively, and an antibody light chain variable region (hereinafter, abbreviated to VL) comprising CDRs 1, 2, and 3 comprising amino acid sequences represented by SEQ ID NOS: 4, 5, and 6, respectively.

5. The preventive or therapeutic agent for HAM according to any one of claims 1 to 4,
wherein the anti-human CCR4 antibody or the antibody fragment thereof is an anti-human CCR4 antibody or an antibody fragment thereof comprising VH comprising an amino acid sequence represented by SEQ ID NO: 7 and VL comprising an amino acid sequence represented by SEQ ID NO: 8.

6. The preventive or therapeutic agent for HAM according to any one of claims 1 to 5,
wherein the anti-human CCR4 antibody is mogamulizumab.

7. The preventive or therapeutic agent for HAM according to any one of claims 1 to 6,
wherein the antibody fragment is Fab, Fab', $F(ab')_2$, scFv, or a CDR-containing peptide.

8. The preventive or therapeutic agent for HAM according to any one of claims 1 to 7,
wherein an immunosuppressant is used in combination.

9. The preventive or therapeutic agent for HAM according to claim 8,
wherein the immunosuppressant is administered at a low dose.

10. The preventive or therapeutic agent for HAM according to claim 8 or 9,
wherein the immunosuppressant is any one immunosuppressant selected from prednisolone, methylprednisolone, dexamethasone, betamethasone, azathioprine, cyclosporine, tacrolimus, a JAK inhibitor, and an NF-κB inhibitor.

11. The preventive or therapeutic agent for HAM according to any one of claims 1 to 10,
wherein the preventive or therapeutic agent decreases at least any one biomarker selected from the amount of HTLV-1 proviral DNA in the peripheral blood, the amount of HTLV-1 proviral DNA in the cerebrospinal fluid (hereinafter abbreviated to CSF), and the number of cells in CSF of a HAM patient.

12. The preventive or therapeutic agent for HAM according to any one of claims 1 to 11,
wherein the preventive or therapeutic agent decreases the amount of at least one of neopterin and CXCL10 in CSF of a HAM patient.

13. The preventive or therapeutic agent for HAM according to any one of claims 1 to 12,
wherein the preventive or therapeutic agent prevents deterioration of the motor function of a HAM patient or improves the motor function.

14. The preventive or therapeutic agent for HAM according to claim 13,
wherein the motor function is evaluated by at least one of Modified Ashworth Scale (hereinafter abbreviated to MAS) and Osame's motor disability score (hereinafter abbreviated to OMDS).

15. A preventive or therapeutic method for HAM, comprising:
administering an anti-human CCR4 antibody or an antibody fragment thereof at a low dose.

16. The preventive or therapeutic method for HAM according to claim 15, comprising:
administering the anti-human CCR4 antibody or the antibody fragment thereof at a dose of 1 mg/kg or less at administration intervals of 4 weeks or more.

17. The preventive or therapeutic method for HAM according to claim 15 or 16, comprising:
administering the anti-human CCR4 antibody or the antibody fragment thereof at a dose of 0.3 mg/kg at administration intervals of 12 weeks.

18. The preventive or therapeutic method for HAM according to any one of claims 15 to 17,
wherein the anti-human CCR4 antibody or the antibody fragment thereof is an anti-human CCR4 antibody or an antibody fragment thereof including VH comprising CDRs 1, 2, and 3 comprising amino acid sequences represented by SEQ ID NOS: 1, 2, and 3, respectively, and VL containing CDRs 1, 2, and 3 comprising amino acid sequences represented by SEQ ID NOS: 4, 5, and 6, respectively.

19. The preventive or therapeutic method for HAM according to any one of claims 15 to 18,
    wherein the anti-human CCR4 antibody or the antibody fragment thereof is an anti-human CCR4 antibody or an antibody fragment thereof comprising VH comprising an amino acid sequence represented by SEQ ID NO: 7 and VL comprising an amino acid sequence represented by SEQ ID NO: 8.

20. The preventive or therapeutic method for HAM according to any one of claims 15 to 19,
    wherein the anti-human CCR4 antibody is mogamulizumab.

21. The preventive or therapeutic method for HAM according to any one of claims 15 to 20,
    wherein the antibody fragment is Fab, Fab', $F(ab')_2$, scFv, or a CDR-containing peptide.

22. The preventive or therapeutic method for HAM according to any one of claims 15 to 21, comprising:
    using an immunosuppressant in combination.

23. The preventive or therapeutic method for HAM according to claim 22, comprising:
    administering the immunosuppressant at a low dose.

24. The preventive or therapeutic method for HAM according to claim 22 or 23,
    wherein the immunosuppressant is any one immunosuppressant selected from prednisolone, methylprednisolone, dexamethasone, betamethasone, azathioprine, cyclosporine, tacrolimus, a JAK inhibitor, and an NF-κB inhibitor.

25. The preventive or therapeutic method for HAM according to any one of claims 15 to 24,
    wherein the preventive or therapeutic method decreases at least any one biomarker selected from the amount of HTLV-1 proviral DNA in the peripheral blood, the amount of HTLV-1 proviral DNA in CSF, and the number of cells in CSF of a HAM patient.

26. The preventive or therapeutic method for HAM according to any one of claims 15 to 25,
    wherein the preventive or therapeutic method decreases the amount of at least one of neopterin and CXCL10 in CSF of a HAM patient.

27. The preventive or therapeutic method for HAM according to any one of claims 15 to 26,
    wherein the preventive or therapeutic method prevents deterioration of the motor function of a HAM patient or improves the motor function.

28. The preventive or therapeutic method for HAM according to claim 27,
    wherein the motor function is evaluated by at least one of MAS and OMDS.

29. An anti-human CCR4 antibody or an antibody fragment thereof for use in treatment or prevention for HAM,
    wherein the antibody or the antibody fragment thereof is administered at a low dose.

30. Use of an anti-human CCR4 antibody or an antibody fragment thereof for producing a preventive agent or a therapeutic agent for HAM,
    wherein the preventive agent or the therapeutic agent is administered at a low dose.

31. A method for preventing deterioration of the motor function of a HAM patient and/or a method for improving the motor function, comprising:
    administering an anti-human CCR4 antibody or an antibody fragment thereof at a low dose.

32. The method for preventing deterioration of the motor function of a HAM patient and/or the method for improving the motor function according to claim 31, comprising:
    administering the anti-human CCR4 antibody or the antibody fragment thereof at a dose of 1 mg/kg or less at administration intervals of 4 weeks or more.

33. The method for preventing deterioration of the motor function of a HAM patient and/or the method for improving the motor function according to claim 31 or 32, comprising:
    administering the anti-human CCR4 antibody or the antibody fragment thereof at a dose of 0.3 mg/kg at administration intervals of 12 weeks.

34. The method for preventing deterioration of the motor function of a HAM patient and/or the method for improving the motor function according to any one of claims 31 to 33, wherein the anti-human CCR4 antibody or the antibody fragment thereof is an anti-human CCR4 antibody or an antibody fragment thereof including VH containing CDRs 1, 2, and 3 comprising amino acid sequences represented by SEQ ID NOS: 1, 2, and 3, respectively, and VL comprising CDRs 1, 2, and 3 comprising amino acid sequences represented by SEQ ID NOS: 4, 5, and 6, respectively.

35. The method for preventing deterioration of the motor function of a HAM patient and/or the method for improving the motor function according to any one of claims 31 to 34, wherein the anti-human CCR4 antibody or the antibody fragment thereof is an anti-human CCR4 antibody or an antibody fragment thereof comprising VH comprising an amino acid sequence represented by SEQ ID NO: 7 and VL comprising an amino acid sequence represented by SEQ ID NO: 8.

36. The method for preventing deterioration of the motor function of a HAM patient and/or the method for improving the motor function according to any one of claims 31 to 35, wherein the anti-human CCR4 antibody is mogamulizumab.

37. The method for preventing deterioration of the motor function of a HAM patient and/or the method for improving the motor function according to any one of claims 31 to 36, wherein the antibody fragment is Fab, Fab', $F(ab')_2$, scFv, or a CDR-containing peptide.

38. The method for preventing deterioration of the motor function of a HAM patient and/or the method for improving the motor function according to any one of claims 31 to 37, comprising: using an immunosuppressant in combination.

39. The method for preventing deterioration of the motor function of a HAM patient and/or the method for improving the motor function according to claim 38, comprising: administering the immunosuppressant at a low dose.

40. The method for preventing deterioration of the motor function of a HAM patient and/or the method for improving the motor function according to claim 38 or 39, wherein the immunosuppressant is any one immunosuppressant selected from prednisolone, methylprednisolone, dexamethasone, betamethasone, azathioprine, cyclosporine, tacrolimus, a JAK inhibitor, and an NF-κB inhibitor.

41. The method for preventing deterioration of the motor function of a HAM patient and/or the method for improving the motor function according to any one of claims 31 to 40, wherein the method decreases at least any one biomarker selected from the amount of HTLV-1 proviral DNA in the peripheral blood, the amount of HTLV-1 proviral DNA in CSF, and the number of cells in CSF of a HAM patient.

42. The method for preventing deterioration of the motor function of a HAM patient and/or the method for improving the motor function according to any one of claims 31 to 41, wherein the method decreases the amount of at least one of neopterin and CXCL1 0 in CSF of a HAM patient.

FIG. 1

FIG. 2

FIG. 3

FIG. 4

FIG. 5

## FIG. 6

FIG. 7

# FIG. 8

*FIG. 9*

FIG. 10

*FIG. 11*

*FIG. 12*

*FIG. 13*

TRIAL PERIOD (DAY)

GRADE 1
GRADE 2
GRADE 3
GRADE 4
GRADE 5
GRADE 6

*FIG. 14*

TRIAL PERIOD (MONTH)

GRADE 1
GRADE 2
GRADE 3
GRADE 4
GRADE 5
GRADE 6

## FIG. 15

## INTERNATIONAL SEARCH REPORT

International application No.

PCT/JP2018/008166

### A. CLASSIFICATION OF SUBJECT MATTER

Int.Cl. A61K39/395(2006.01)i, A61K31/52(2006.01)i, A61K31/573(2006.01)i,
A61K31/706(2006.01)i, A61K38/13(2006.01)i, A61K45/00(2006.01)i,
A61P19/00(2006.01)i, A61P31/12(2006.01)i, A61P35/02(2006.01)i,
A61P37/06(2006.01)i, A61P43/00(2006.01)i

According to International Patent Classification (IPC) or to both national classification and IPC

### B. FIELDS SEARCHED

Minimum documentation searched (classification system followed by classification symbols)

Int.Cl. A61K39/395, A61K31/52, A61K31/573, A61K31/706, A61K38/13,
A61K45/00, A61P19/00, A61P31/12, A61P35/02, A61P37/06, A61P43/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

WPI, JSTPlus/JMEDPlus/JST7580 (JDreamIII), CAplus/MEDLINE/EMBASE/BIOSIS
(STN), UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| X<br>Y<br><br>A | ISHIDA, T. et al., "Defucosy lated anti-CCR4 monoclonal antibody (KW-0761) for relapsed adult T-cell leukemia-lymphoma: a multicenter phase II study.", J. Clin. Oncol., 2012, vol. 30, no. 8, pp. 837-42, abstract, page 838, left column, last paragraph | 29<br>1, 2, 4-16, 18-28, 30-32, 34-42<br>3, 17, 33 |
| X<br>Y<br><br><br>A | OGURA, M. et al., "Multicenter phase II study of mogamulizumab (KW-0761), a defucosylated anti-CC chemokine receptor 4 antibody, in patients with relapsed peripheral T-cell lymphoma and cutaneous T-cell lymphoma.", J. Clin. Oncol., 2014, vol. 32, no. 11, pp. 1157-63, abstract, page 1158, left column, last paragraph | 29<br>1, 2, 4-16, 18-28, 30-32, 34-42<br>3, 17, 33 |

☒ Further documents are listed in the continuation of Box C. ☐ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15 May 2018 (15.05.2018) | 29 May 2018 (29.05.2018) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

**INTERNATIONAL SEARCH REPORT**

| International application No. |
| --- |
| PCT/JP2018/008166 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y<br><br>A | WO 2014/07303 A1 (ST. MARIANNA UNIVERSITY SCHOOL OF MEDICINE, KYOWA HAKKO KIRIN COMPANY, LIMITED) 09 January 2014, claims 1, 7-9, paragraphs [0168], [0169] & US 2014/0037654 A1 & EP 2870973 A1, claims 1, 7-9, paragraphs [0172], [0173] & AU 2013285970 A & CA 2877848 A & MX 2015000254 A | 1, 2, 4-16, 18-28, 30-32, 34-42<br>3, 17, 33 |
| Y<br><br>A | 山野嘉久，HAM に対するヒト化抗 CCR4 抗体の医師主導治験，臨床評価，2016, vol. 43, no. 2, pp. 418-421, page 419, right column, column 4, (Clinical Evaluation), non-official translation (YAMANO, Yoshihisa, "Doctor-led clinical trial of humanized anti-CCR4 antibody for HAM") | 1, 2, 4-16, 18-28, 30-32, 34-42<br>3, 17, 33 |
| Y<br><br>A | 新谷奈津美他，HTLV-1 関連脊髄症(HAM)の分子病態解明による治療薬開発の新展開，日本臨床免疫学会会誌，2016, vol. 39, no. 3, pp. 207-212, abstract, page 211, left column, line 12 to right column, line 6, (ARAYA, Natsumi et al., "Developing novel treatments for HTLV-1-associated myelopathy (HAM) by investigating molecular pathomechanisms", Japanese journal of clinical immunology) | 1, 2, 4-16, 18-28, 30-32, 34-42<br>3, 17, 33 |
| P, X | 山野嘉久，HTLV-1 関連脊髄症に対するヒト化抗 CCR4 抗体療法開発の背景，神経治療，February 2018, vol. 34, no. 4, pp. 453-457, (YAMANO, Yoshihisa, "The background to develop a humanized anti CCR4 antibody as a treatment for HAM", Neurological Therapeutics) | 1-42 |
| P, X | 新谷奈津美他，成人 T 細胞白血病リンパ腫(ATL)研究と診療の進歩 HTLV-1 関連脊髄症(HAM)の分子病態と治療，血液内科，March 2017, vol. 74, no. 3, pp. 373-379, (Hematology), non-official translation (ARAYA, Natsumi et al., "Advancement in research and treatment of adult T-cell leukemia/lymphoma (ATL): Molecular pathogenesis and treatment of HTLV-1 associated myelopathy (HAM)") | 1-42 |
| P, X | 福島伯泰他，HTLV-1 関連疾患の診断と治療の進歩 VIII. HTLV-1 関連疾患に対する新規治療法の展望，日本内科学会雑誌，July 2017, vol. 106, no. 7, pp. 1417-1422, (The Journal of the Japanese Society of Internal Medicine), non-official translation (FUKUSHIMA, Noriyasu et al., "Advancement in diagnosis and treatment of HTLV-1 associated disease: VIII. Future prospects for novel treatment of HTLV-1 associated diseases") | 1-42 |
| A | YAMAUCHI, J. et al., "Mogamulizumab, an anti-CCR4 antibody, targets human T-lymphotropic virus type 1-infected CD8+ and CD4+ T cells to treat associated myelopathy.", J. Infect. Dis., 2015, vol. 211, no. 2, pp. 238-48 | 1-42 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010017130 A
- JP 2010100578 A
- WO 2014007303 A
- WO 2005035586 A
- WO 200231140 A
- WO 0061739 A

- US 20070148165
- US 6737056 B
- US 7297775 B
- US 7317091 B
- WO 2005070963 A
- JP 2017039506 A

**Non-patent literature cited in the description**

- **UCHIYAMA et al.** *Blood,* 1977, vol. 50, 481-492
- **GESSAIN et al.** *Lancet,* 1985, vol. 2, 407-410
- **OSAME et al.** *Lancet,* 1986, vol. 1, 1031-1032
- **KAPLAN et al.** *J. Aquir. Immune Defi. Syndro.,* 1990, vol. 3, 1096-1101
- **FUZII et al.** *Life Sciences,* 2014, vol. 104, 9-14
- **NAKAGAWA et al.** *J. Neurovirol.,* 1995, vol. 1, 50-61
- **YAMANO et al.** *The Journal of Clinical Investigation,* 2005, vol. 115, 1361-1368
- **OLIND et al.** *Arch. Neurol.,* 2006, vol. 63, 1560-1566
- **YAMANO et al.** *PLoS One,* 2009, vol. 4, e6517
- **ARAYA et al.** *Viruses,* 2011, vol. 3, 1532-1548
- **YAMANO et al.** *Clinical and Experomental Neuroim-munology,* 2015, vol. 6, 395-401
- **NIWA et al.** *Cancer Res.,* 2004, vol. 64, 2127-2133
- **ISHII et al.** *Clin. Cancer Res.,* 2010, vol. 16, 1520-1531
- **D. PODSIADLO et al.** *Journal of American Geriatrics Society,* 1991, vol. 39, 142-148
- **R. W. BOHANNON et al.** *Physigcal Therapy,* 1987, vol. 67 (2), 206-207

- **S. IZUMO et al.** *Neurology,* 1996, vol. 46 (4), 1016-1021
- **YAMANO et al.** *Blood,* 2002, vol. 99, 88-94
- **YAMANO et al.** *J. Exp. Med.,* 2004, vol. 199, 1367-1377
- **YAMANO et al.** *J. Clin Invest.,* 2005, vol. 115, 1361-1368
- **YAMANO et al.** *Blood,* 2002, vol. 99 (1), 88-94
- **SATO et al.** *PLOS Neglected Tropical Disease,* 2013, vol. 7 (10), e2479
- **SATO et al.** *PLOS Neglected Tropical Diseases,* 2013, vol. 7 (10), e2479
- *J. Biol. Chem.,* 1996, vol. 271, 21514
- *J. Exp. Med.,* 1997, vol. 185, 1595
- *J. Biol. Chem.,* 1997, vol. 272, 25229
- **KABAT et al.** *Sequence of Proteins of immunological interests,* 1991
- **KOBAYASHI et al.** *Clinical Cancer Research,* 2014, vol. 20 (11), 2851-61
- Review of WHO Kagoshima Meeting and Diagnostic Guidelines for HAM/TSP. **OSAME M.** Human Retro-virology. Raven Press, Ltd, 1990, 191-7
- **YAMANO et al.** *Clinical and Experimental Neuroim-munology,* 2015, vol. 6, 395-401
- **ISHIDA et al.** *J. Clin. Oncol.,* 2012, vol. 30 (8), 837-842

SEQUENCE LISTING

<110> ST.MARIANNA UNIVERSITY SCHOOL OF MEDICINE
Kyowa Hakko Kirin,Co.,Ltd.

<120> The preventive or therapeutic medicament for HTLV-1 associated
myelopathy using low dose of anti-CCR4 antibody

<130> W524404

<150> JP2017-039506
<151> 2017-03-02

<160> 9

<170> PatentIn version 3.3

<210> 1
<211> 5
<212> PRT
<213> Artificial Sequence

<220>
<223> Description of the artificial sequence;anti-human CCR4 antibody H
chain CDR1 amino acid sequence

<400> 1

Asn Tyr Gly Met Ser
1               5

<210> 2
<211> 17
<212> PRT
<213> Artificial Sequence

<220>
<223> Description of the artificial sequence;anti-human CCR4 antibody H
chain CDR2 amino acid sequence

<400> 2

Thr Ile Ser Ser Ala Ser Thr Tyr Ser Tyr Tyr Pro Asp Ser Val Lys
1               5                   10                  15

Gly

<210> 3
<211> 10
<212> PRT
<213> Artificial Sequence

<220>
<223> Description of the artificial sequence;anti-human CCR4 antibody H
chain CDR3 amino acid sequence

<400> 3

His Ser Asp Gly Asn Phe Ala Phe Gly Tyr

<210> 4
<211> 16
<212> PRT
<213> Artificial Sequence

<220>
<223> Description of the artificial sequence;anti-human CCR4 antibody L chain CDR1 amino acid sequence

<400> 4

Arg Ser Ser Arg Asn Ile Val His Ile Asn Gly Asp Thr Tyr Leu Glu
1               5                   10                  15


<210> 5
<211> 7
<212> PRT
<213> Artificial Sequence

<220>
<223> Description of the artificial sequence;anti-human CCR4 antibody L chain CDR2 amino acid sequence

<400> 5

Lys Val Ser Asn Arg Phe Ser
1               5


<210> 6
<211> 9
<212> PRT
<213> Artificial Sequence

<220>
<223> Description of the artificial sequence;anti-human CCR4 antibody L chain CDR3 amino acid sequence

<400> 6

Phe Gln Gly Ser Leu Leu Pro Trp Thr
1               5


<210> 7
<211> 119
<212> PRT
<213> Artificial Sequence

<220>
<223> Description of the artificial sequence;anti-human CCR4 antibody VH amino acid sequence

<400> 7

Glu Val Gln Leu Val Glu Ser Gly Gly Asp Leu Val Gln Pro Gly Arg
1               5                   10                  15

```
Ser Leu Arg Leu Ser Cys Ala Ala Ser Gly Phe Ile Phe Ser Asn Tyr
            20              25                  30

Gly Met Ser Trp Val Arg Gln Ala Pro Gly Lys Gly Leu Glu Trp Val
            35              40                  45

Ala Thr Ile Ser Ser Ala Ser Thr Tyr Ser Tyr Tyr Pro Asp Ser Val
            50              55                  60

Lys Gly Arg Phe Thr Ile Ser Arg Asp Asn Ala Lys Asn Ser Leu Tyr
65              70              75                  80

Leu Gln Met Asn Ser Leu Arg Val Glu Asp Thr Ala Leu Tyr Tyr Cys
                85              90                  95

Gly Arg His Ser Asp Gly Asn Phe Ala Phe Gly Tyr Trp Gly Gln Gly
                100             105                 110

Thr Leu Val Thr Val Ser Ser
                115


<210>  8
<211>  112
<212>  PRT
<213>  Artificial Sequence

<220>
<223>  Description of the artificial sequence;anti-human CCR4 antibody VL
amino acid sequence

<400>  8

Asp Val Leu Met Thr Gln Ser Pro Leu Ser Leu Pro Val Thr Pro Gly
1               5               10                  15

Glu Pro Ala Ser Ile Ser Cys Arg Ser Ser Arg Asn Ile Val His Ile
            20              25                  30

Asn Gly Asp Thr Tyr Leu Glu Trp Tyr Leu Gln Lys Pro Gly Gln Ser
            35              40                  45

Pro Gln Leu Leu Ile Tyr Lys Val Ser Asn Arg Phe Ser Gly Val Pro
            50              55                  60

Asp Arg Phe Ser Gly Ser Gly Ser Gly Thr Asp Phe Thr Leu Lys Ile
65              70              75                  80

Ser Arg Val Glu Ala Glu Asp Val Gly Val Tyr Tyr Cys Phe Gln Gly
                85              90                  95
```

```
Ser Leu Leu Pro Trp Thr Phe Gly Gln Gly Thr Lys Val Glu Ile Lys
            100                 105                 110
```

<210> 9
<211> 360
<212> PRT
<213> Homo sapiens

<400> 9

```
Met Asn Pro Thr Asp Ile Ala Asp Thr Thr Leu Asp Glu Ser Ile Tyr
1               5                   10                  15

Ser Asn Tyr Tyr Leu Tyr Glu Ser Ile Pro Lys Pro Cys Thr Lys Glu
            20                  25                  30

Gly Ile Lys Ala Phe Gly Glu Leu Phe Leu Pro Pro Leu Tyr Ser Leu
            35                  40                  45

Val Phe Val Phe Gly Leu Leu Gly Asn Ser Val Val Val Leu Val Leu
    50                  55                  60

Phe Lys Tyr Lys Arg Leu Arg Ser Met Thr Asp Val Tyr Leu Leu Asn
65                  70                  75                  80

Leu Ala Ile Ser Asp Leu Leu Phe Val Phe Ser Leu Pro Phe Trp Gly
            85                  90                  95

Tyr Tyr Ala Ala Asp Gln Trp Val Phe Gly Leu Gly Leu Cys Lys Met
            100                 105                 110

Ile Ser Trp Met Tyr Leu Val Gly Phe Tyr Ser Gly Ile Phe Phe Val
            115                 120                 125

Met Leu Met Ser Ile Asp Arg Tyr Leu Ala Ile Val His Ala Val Phe
            130                 135                 140

Ser Leu Arg Ala Arg Thr Leu Thr Tyr Gly Val Ile Thr Ser Leu Ala
145                 150                 155                 160

Thr Trp Ser Val Ala Val Phe Ala Ser Leu Pro Gly Phe Leu Phe Ser
            165                 170                 175

Thr Cys Tyr Thr Glu Arg Asn His Thr Tyr Cys Lys Thr Lys Tyr Ser
            180                 185                 190

Leu Asn Ser Thr Thr Trp Lys Val Leu Ser Ser Leu Glu Ile Asn Ile
            195                 200                 205
```

```
Leu Gly Leu Val Ile Pro Leu Gly Ile Met Leu Phe Cys Tyr Ser Met
    210             215             220

Ile Ile Arg Thr Leu Gln His Cys Lys Asn Glu Lys Lys Asn Lys Ala
225             230             235             240

Val Lys Met Ile Phe Ala Val Val Val Leu Phe Leu Gly Phe Trp Thr
            245             250             255

Pro Tyr Asn Ile Val Leu Phe Leu Glu Thr Leu Val Glu Leu Glu Val
            260             265             270

Leu Gln Asp Cys Thr Phe Glu Arg Tyr Leu Asp Tyr Ala Ile Gln Ala
        275             280             285

Thr Glu Thr Leu Ala Phe Val His Cys Cys Leu Asn Pro Ile Ile Tyr
    290             295             300

Phe Phe Leu Gly Glu Lys Phe Arg Lys Tyr Ile Leu Gln Leu Phe Lys
305             310             315             320

Thr Cys Arg Gly Leu Phe Val Leu Cys Gln Tyr Cys Gly Leu Leu Gln
            325             330             335

Ile Tyr Ser Ala Asp Thr Pro Ser Ser Ser Tyr Thr Gln Ser Thr Met
        340             345             350

Asp His Asp Leu His Asp Ala Leu
        355             360
```